(19) Europäisches Patentamt
European Patent Office
Office européen des brevets

(11) **EP 4 283 737 A1**

(12) **EUROPEAN PATENT APPLICATION**
published in accordance with Art. 153(4) EPC


(43) Date of publication:
**29.11.2023 Bulletin 2023/48**

(21) Application number: **21921506.8**

(22) Date of filing: **30.12.2021**

(51) International Patent Classification (IPC):
***H01M 10/0567*** *(2010.01)* ***H01M 4/525*** *(2010.01)*
***H01M 4/505*** *(2010.01)* ***H01M 10/052*** *(2010.01)*

(86) International application number:
**PCT/KR2021/020273**

(87) International publication number:
**WO 2022/158749 (28.07.2022 Gazette 2022/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.01.2021 KR 20210008909**



(71) Applicant: **Samsung SDI Co., Ltd.**
**Yongin-si, Gyeonggi-do 17084 (KR)**

(72) Inventors:
- **PARK, Hong Ryeol**
  **Yongin-si, Gyeonggi-do 17084 (KR)**
- **KIM, Myung Hoon**
  **Yongin-si, Gyeonggi-do 17084 (KR)**
- **KIM, Sang Hoon**
  **Yongin-si, Gyeonggi-do 17084 (KR)**
- **KIM, Yun Hee**
  **Yongin-si, Gyeonggi-do 17084 (KR)**
- **PARK, In Jun**
  **Yongin-si, Gyeonggi-do 17084 (KR)**
- **SHIN, Jeong Min**
  **Yongin-si, Gyeonggi-do 17084 (KR)**
- **OH, Seung Ryong**
  **Yongin-si, Gyeonggi-do 17084 (KR)**
- **JEONG, Myung Hwan**
  **Yongin-si, Gyeonggi-do 17084 (KR)**
- **CHOI, Hyun Bong**
  **Yongin-si, Gyeonggi-do 17084 (KR)**

(74) Representative: **Gulde & Partner**
**Patent- und Rechtsanwaltskanzlei mbB**
**Wallstraße 58/59**
**10179 Berlin (DE)**


(54) **ADDITIVE FOR ELECTROLYTE OF LITHIUM BATTERY, ORGANIC ELECTROLYTE COMPRISING SAME AND LITHIUM BATTERY COMPRISING ELECTROLYTE**


(57) Provided are: an additive for an electrolyte of a lithium battery, the additive including a compound represented by Formula 1 and a compound represented by Formula 2; an organic electrolyte including the additive; and a lithium battery including the electrolyte. Accordingly, lifespan characteristics of the lithium battery may be improved and the amount of gas generation may be reduced.


FIG. 1

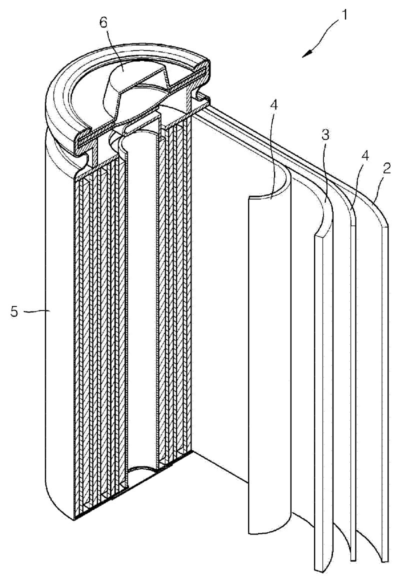


EP 4 283 737 A1

## Description

TECHNICAL FIELD

The disclosure relates to an additive for an electrolyte of a lithium battery, an organic electrolyte including the same, and a lithium battery including the electrolyte.

BACKGROUND ART

Lithium batteries are used as driving power sources for portable electronic devices, including video cameras, mobile phones, and laptop computers. Rechargeable lithium secondary batteries are rechargeable at high rates and have a high energy density per unit weight of at least 3 times higher than that of conventional lead storage batteries, nickel-cadmium batteries, nickel-hydride batteries, nickel-zinc batteries, or the like.
Since lithium batteries operate at high operating voltages, they are not compatible with aqueous electrolytes highly reactive to lithium. Lithium batteries mostly use an organic electrolyte. The organic electrolyte may be prepared by dissolving a lithium salt in an organic solvent. An appropriate organic solvent for the organic electrolyte may be stable at high voltages, and have a high ionic conductivity, a high dielectric constant, and a low viscosity.
When a lithium battery uses a carbonate-based polar nonaqueous solvent, an irreversible side reaction between the electrolyte solution and a positive or negative electrode may occur during an initial charging process, causing excess consumption of charges.
As a result of the irreversible side reaction, a passivation layer such as a solid electrolyte interface layer (hereinbelow, referred to as SEI layer) may be formed on the surface of the negative electrode. The SEI layer may prevent decomposition of the electrolyte solution during charging and discharging processes and act as an ion tunnel. The higher stability and lower resistance the SEI layer has, the better lifespan the lithium battery may have.
In addition, the irreversible side reaction may also result in a protection layer on the surface of the positive electrode. The protection layer may prevent decomposition of the electrolyte solution during charging and discharging processes and act as an ion tunnel. The higher stability the protection layer has at high temperatures, the better lifespan the lithium battery may have.
Various additives are being used for stabilization of the SEI layer and/or the protection layer. However, SEI layers formed using conventional additives in the related art are prone to degradation at high temperatures. That is, such SEI layers and/or protection layers show poor stability at high temperatures.
Therefore, there is a demand for an organic electrolyte capable of forming an SEI and/or a protection layer with improved high-temperature stability.

DESCRIPTION OF EMBODIMENTS5

TECHNICAL PROBLEM

One aspect of the disclosure provides an additive for an electrolyte of a lithium battery.
Another aspect of the disclosure provides an organic electrolyte including the additive.
Another aspect of the disclosure provides a lithium battery including the organic electrolyte.

SOLUTION TO PROBLEM

According to one aspect,
provided is an additive for an electrolyte of a lithium battery, the additive including a compound represented by Formula 1 below and a compound represented by Formula 2 below:

Formula 1

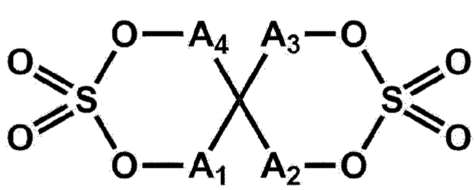

Formula 2

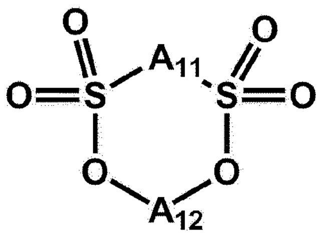

In Formulas 1 and 2,
$A_1$, to $A_4$, $A_{11}$, and $A_{12}$ may be each independently a C1-C5 alkylene group unsubstituted or substituted with a substituent; a carbonyl group; or a sulfinyl group.

According to another aspect,

provided is an organic electrolyte including: a lithium salt;
an organic solvent; and
the above-described additive.

According to another aspect,

provided is a lithium battery including: a positive electrode;
a negative electrode; and
the above-described organic electrolyte.

ADVANTAGEOUS EFFECTS OF DISCLOSURE

According to one aspect, by using an additive for an electrolyte of a lithium battery, which includes two types of compounds, each satisfying a specific structure, a lithium battery using an organic electrolyte containing the additive may have improved lifespan characteristics.

BRIEF DESCRIPTION OF DRAWINGS

FIG. 1 is a schematic diagram of a lithium battery according to an exemplary embodiment.

MODE OF DISCLOSURE

The present inventive concept, which will be more fully described hereinafter, may have various variations and various embodiments, and specific embodiments will be illustrated in the accompanied drawings and described in greater details. However, the present inventive concept should not be construed as being limited to specific embodiments set forth herein. Rather, these embodiments are to be understood as encompassing all variations, equivalents, or alternatives included in the scope of the present inventive concept.

The terminology used hereinbelow is used for the purpose of describing particular embodiments only and is not intended to limit the present inventive concept. As used herein, the singular forms are intended to include the plural forms as well, unless the context clearly indicates otherwise. As used herein, the terms "comprises" and/or "comprising," or "includes" and/or "including" when used in this specification, specify the presence of stated features, regions, integers, steps, operations, elements, components, ingredients, materials, or combinations thereof, but do not preclude the presence or addition of one or more other features, regions, integers, steps, operations, elements, components, ingredients, materials, or combinations thereof. As used herein, "I" may be interpreted as "and", or as "or" depending on the context.

In the drawings, the thicknesses of layers and regions may be exaggerated for clarity of description. Like reference numerals denote like elements throughout the specification. Throughout the specification, when a component, such as a layer, a film, a region, or a plate, is described as being "above" or "on" another component, the component may be directly above the another component, or there may be yet another component therebetween.

Hereinbelow, an additive for an electrolyte of a lithium battery, an organic electrolyte including the additive, and a lithium battery including the electrolyte according to exemplary embodiments will be described in greater detail.

An additive for a lithium secondary battery electrolyte according to an embodiment may include a compound represented by Formula 1 below and a compound represented by Formula 2 below:

Formula 1

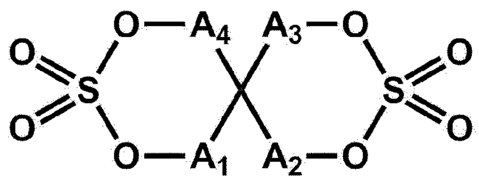

Formula 2

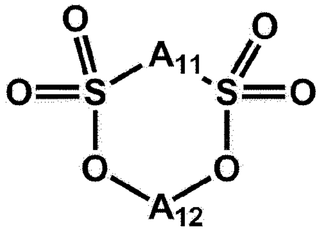

In Formulas 1 and 2,
$A_1$, to $A_4$, $A_{11}$, and $A_{12}$ may be each independently a C1-C5 alkylene group unsubstituted or substituted with a substituent; a carbonyl group; or a sulfinyl group.

By adding an additive including a compound represented by Formula 1 and Formula 2 to an electrolyte of a lithium battery, an improvement in battery performance of the lithium battery such as lifespan characteristics may be achieved, and resistance changes and the amount of gas generation at a high temperature may be reduced.

The compound represented by Formula 1 may have a structure in which two sulfate rings are linked in a spiro form. The compound represented by Formula 2 may have a ring structure that contains two sulfonate groups.

Without limiting the scope of the present disclosure and for better understanding, the mechanism by which the performance of a lithium battery is improved by inclusion of the compound represented by Formula 1 and the compound represented by Formula 2 in an electrolyte will be described in greater detail below.

The sulfate ester groups included in the compound represented by Formula 1 and the compound represented by Formula 2 may be reduced themselves by taking electrons from the surface of the negative electrode during a charging process, or may react with already reduced polar solvent molecules, to thereby influence the properties of the SEI layer being formed on the surface of the negative electrode. For example, the compound represented by Formula 1 and the compound represented by Formula 2, which include the sulfate ester groups, may take electrons from the negative electrode more easily than the polar solvent. That is, the compound represented by Formula 1 and the compound represented by Formula 2 may be reduced at a lower voltage than the polar solvent and thus, may be reduced before the polar solvent is reduced.

For example, the compound represented by Formula 1 and the compound represented by Formula 2 due to containing sulfate ester groups, may be more easily reduced and/or decomposed into radicals and/or ions during charge. Accordingly, the radicals and/or ions may form an SEI layer suitable for a negative electrode by binding to lithium ions, and thus inhibit the formation of additional decomposition products of a solvent.

For example, the compound represented by Formula 1 and the compound represented by Formula 2, for example, may form covalent bonds with various functional groups present on the surface of a carbon-based negative electrode, or with the carbon-based negative electrode, or may be adsorbed to the surface of the electrode. In this case, the compound represented by Formula 2, which has a relatively smaller molecular weight, may form covalent bonds with the carbon-based negative electrode or various functional groups present on the surface of the carbon-based negative electrode, or may be adsorbed to the electrode surface before the compound represented by Formula 1, and may promote the bonding between the compound represented by Formula 1 and various functional groups present on the surface of the carbon-based negative electrode or the carbon-based negative electrode. Such interactions between the compound represented by Formula 1 and the compound represented by Formula 2 may allow the formation of a modified SEI layer with improved stability that remains solid even after prolonged cycling, compared to an SEI layer formed by using only one of the two compounds. In addition, this solid modified SEI layer may more effectively prevent the organic solvent with solvated lithium ions from entering into an electrode during intercalation of lithium ions. Accordingly, since the modified SEI layer more effectively prevents a direct contact between the negative electrode and the organic solvent, reversibility of adsorption/desorption of lithium ions may be further improved, which consequently may lead to an improvement in the discharge capacity and lifespan characteristics of the battery.

In addition, the compound represented by Formula 1 and the compound represented by Formula 2, due to containing sulfate ester groups, may be coordinated with the surface of a positive electrode, and therefore may influence the properties of the protection layer formed on the surface of the positive electrode. For example, the sulfate ester groups in the compound represented by Formula 1 and the compound represented by Formula 2 may be coordinated with transition metal ions of positive electrode active material to form a complex. In this case, the compound represented by Formula 2, which has a relatively smaller molecule size, positioned between the compounds represented by Formula 1 having a relatively larger molecule size, may be coordinated with the transition metal ions of positive electrode active material to form a complex. This solid complex may form a modified protection layer with improved stability that proves more durable even to prolonged cycling, than a protection layer formed by an organic solvent alone. In addition, this stable modified protection layer may more effectively block the organic solvent solvating lithium ions from being cointercalated into the electrode during intercalation of lithium ions. Accordingly, since the modified protection layer more effectively prevents a direct contact between the organic solvent and the positive electrode, reversibility of adsorption/desorption of lithium ions may be further improved, which consequently may lead to an improvement in the stability and lifespan characteristics of the battery.

In addition, compared to common sulfate-based compounds, the compound represented by Formula 1 due to having a plurality of rings linked to each other in a spiro form, may have a relatively larger molecular weight and therefore may be thermally stable. In addition, the compound represented by Formula 2, due to having a smaller size than that of the compound represented by Formula 1, is arranged between the compounds represented by Formula 1 and may more stably form the modified SEI layer and modified protection layer formed by the compound represented by Formula 1 and the compound represented by Formula 2, thus improving blocking properties.

In addition, the compound represented by Formula 2 due to containing two sulfonate groups in a single ring, may provide relatively superior electrical conductivity.

Consequently, the compound represented by Formula 1 and the compound represented by Formula 2 may form an SEI layer on the surface of a negative electrode or a protection layer on the surface of a positive electrode. Further, due to having improved thermal stability and blocking properties, the lifespan characteristics of a lithium battery at a high temperature may be improved, and gas generation at a high temperature may be prevented. In addition, resistance increases at a high temperature may be reduced by the compound represented by Formula 2.

In the compound represented by Formula 1 and the compound represented by Formula 2, at least one from among $A_1$ to $A_4$, $A_{11}$ and $A_{12}$ may be an unsubstituted C1-C5 alkylene group or a substituted C1-C5 alkylene group, wherein the substituent of the substituted C1-C5 alkylene group may be a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C20 cycloalkenyl group unsubstituted or substituted with a halogen atom; a C3-C20 heterocyclyl group unsubstituted or substituted with a halogen atom; a C6-C40 aryl group unsubstituted or substituted with a halogen atom; a C2-C40 heteroaryl group unsubstituted or substituted with a halogen atom; or a polar functional group including a heteroatom.

For example, the substituent may be at least one selected from among a halogen atom, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

For example, in the compound represented by Formula 1 and the compound represented by Formula 2, the substituent of the alkylene group may be a polar functional group containing a heteroatom, and the heteroatom of the polar functional group may be at least one selected from among oxygen, nitrogen, phosphorus, sulfur, silicon, and boron.

For example, the substituted C1-C5 alkylene group may be substituted with a polar group having at least one heteroatom, and the polar group containing the heteroatom may include at least one selected from among -F, -Cl, -Br, -I, $-C(=O)OR^{16}$, $-OC(=O)R^{16}$, $-OR^{16}$, $-OG(=O)OR^{16}$, $-R^{15}OC(=O)OR^{16}$, $-C(=O)R^{16}$, $-R^{15}C(=O)R^{16}$, $-OC(=O)R^{16}$, $-R^{15}OC(=O)R^{16}$, $-(R^{15}O)_k-OR^{16}$, $-(OR^{15})_k-OR^{16}$, $-C(=O)-O-C(=O)R^{16}$, $-R^{15}C(=O)-O-C(=O)R^{16}$, $-SR^{16}$, $-R^{15}SR^{16}$, $-SSR^{16}$, $-R^{15}SSR^{16}$, $-S(=O)R^{16}$, $-R^{15}S(=O)R^{16}$, $-R^{15}C(=S)R^{16}$, $-R^{15}C(=S)SR^{16}$, $-R^{15}SO_3R^{16}$, $-SO_3R^{16}$, $-NNC(=S)R^{16}$, $-R^{15}NNC(=S)R^{16}$, $-R^{15}N=C=S$, -NCO, $-R^{15}-NCO$, $-NO_2$, $-R^{15}NO_2$, $-R^{15}SO_2R^{16}$, $-SO_2R^{16}$,

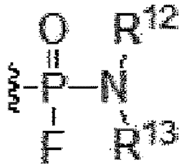

,

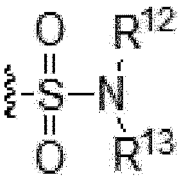 , 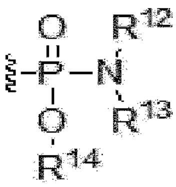 , 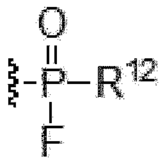 , 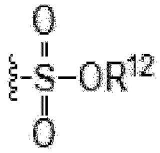 , 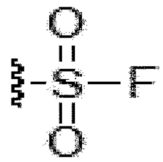 ,

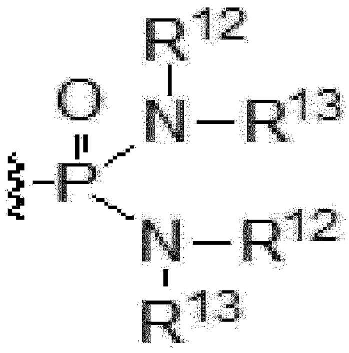 , 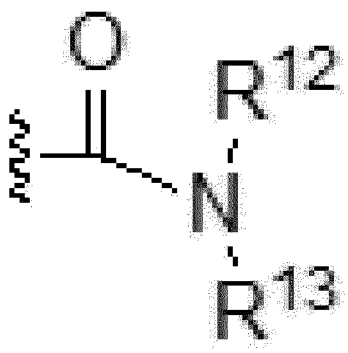 , 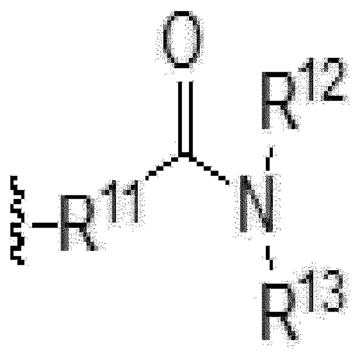 ,

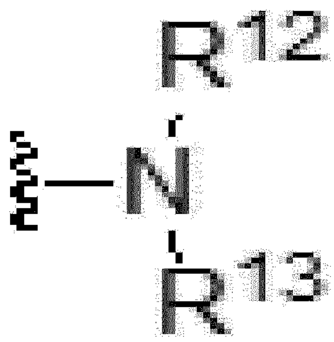 , 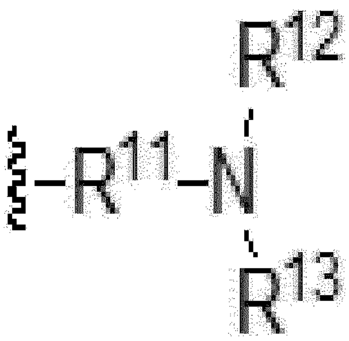 , 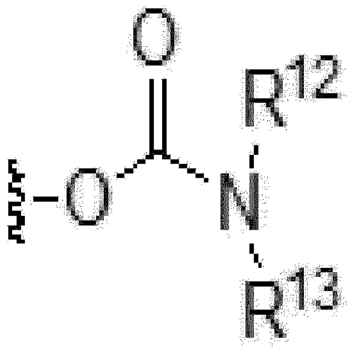 ,

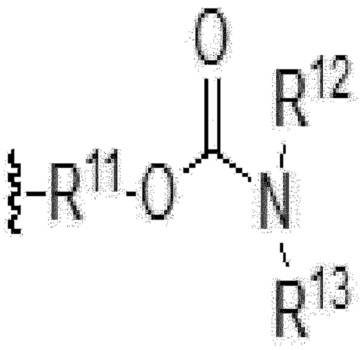 , 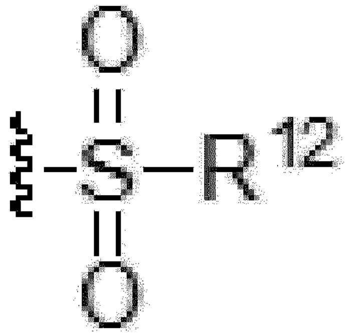 , 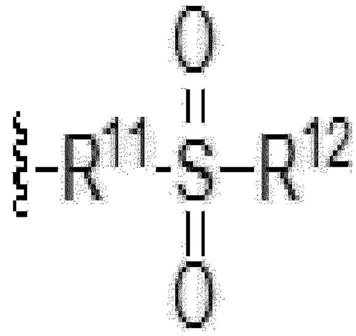 ,

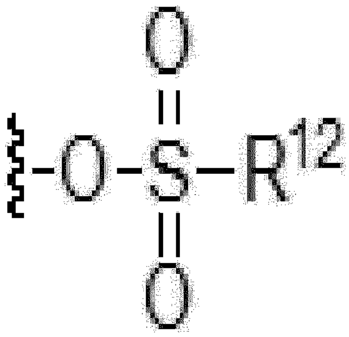,
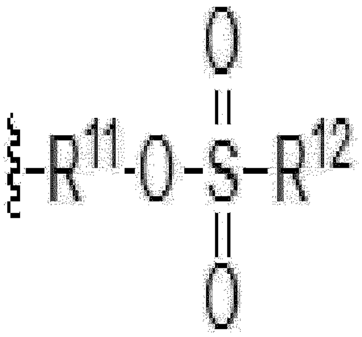,
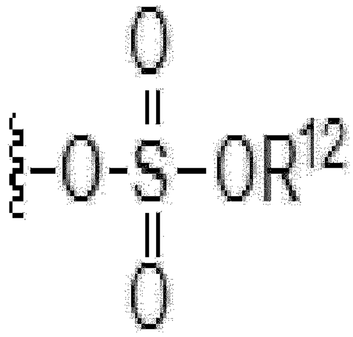,

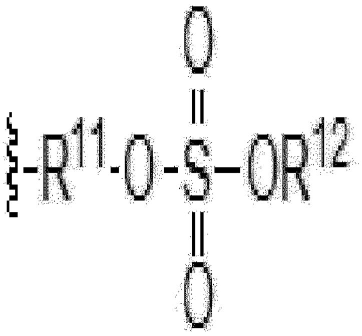,
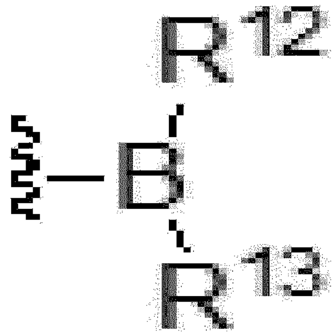,
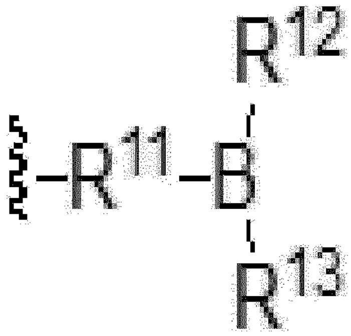,

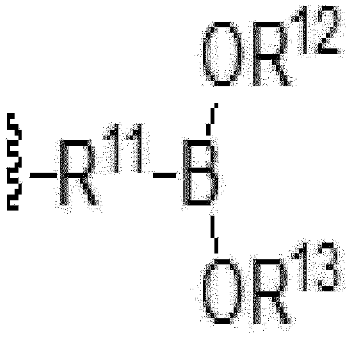,
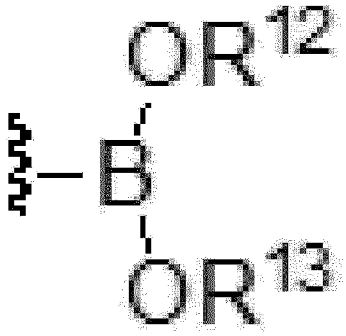,
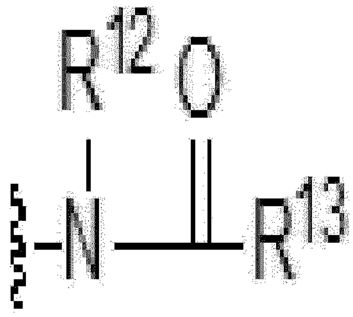,

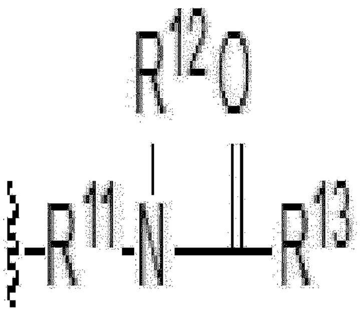,
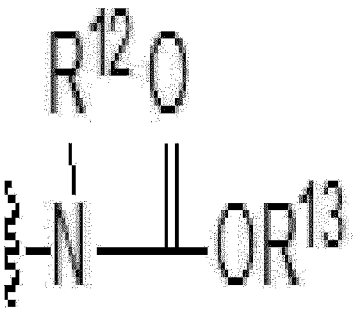,
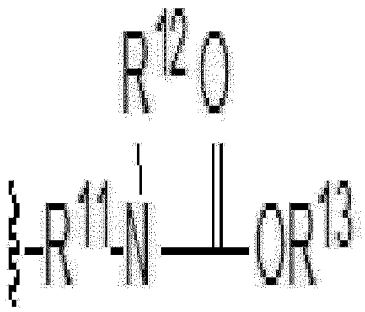,

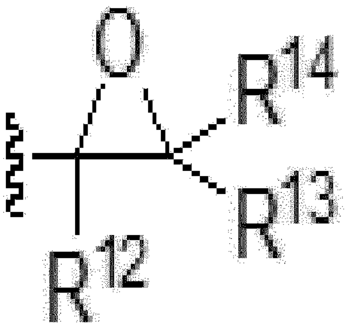,
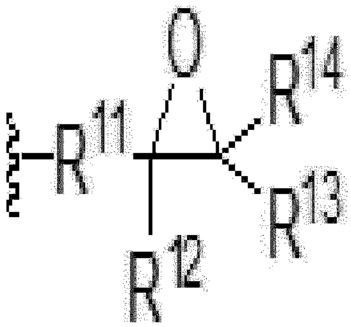,
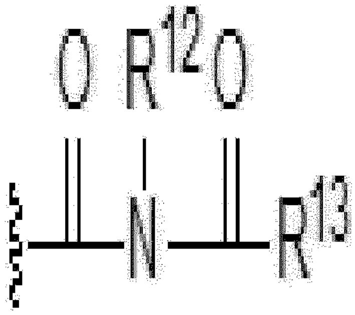,
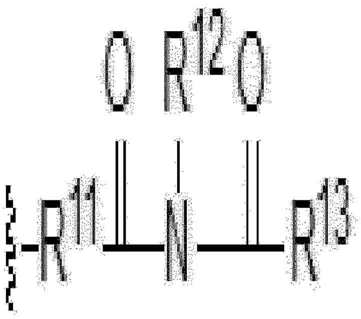,
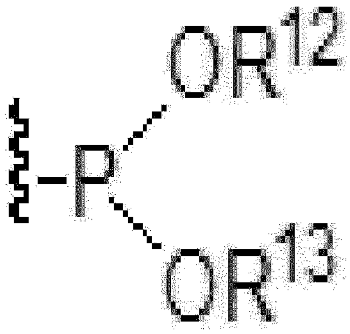,
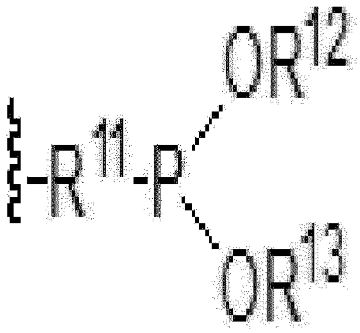,
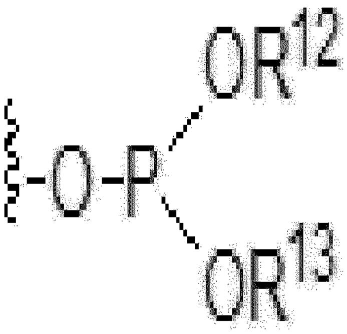,
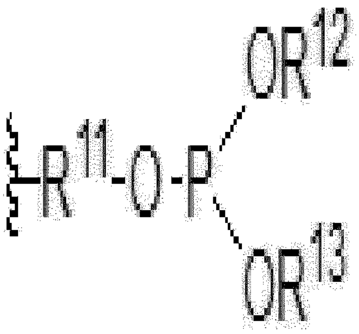,
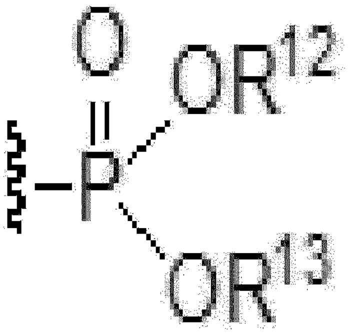,
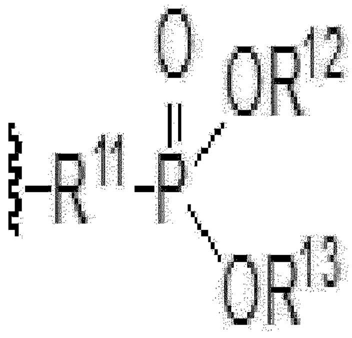,
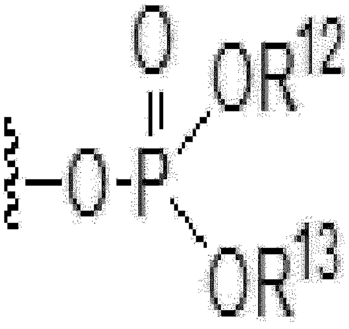, and
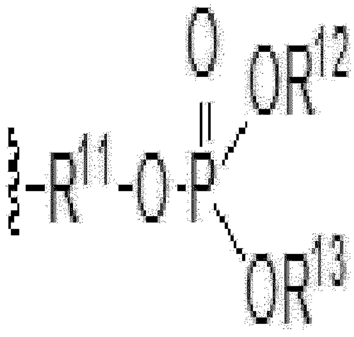,

$R^{11}$ and $R^{15}$ may be each independently a C1-C20 alkylene group unsubstituted or substituted with a halogen atom; a C2-C20 alkenylene group unsubstituted or substituted with a halogen atom; a C2-C20 alkynylene group unsubstituted or substituted with a halogen atom; a C3-C12 cycloalkylene group unsubstituted or substituted with a halogen atom; a C6-C40 arylene group unsubstituted or substituted with a halogen atom; a C2-C40 heteroarylene group unsubstituted or substituted with a halogen atom; a C7-C15 alkylarylene group unsubstituted or substituted with a halogen atom; or a C7-C15 aralkylene group unsubstituted or substituted with a halogen atom,

$R^{12}$, $R^{13}$, $R^{14}$, and $R^{16}$ may be each independently hydrogen; a halogen atom; a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C12 cycloalkyl group unsubstituted or substituted with a halogen atom; a C6-C40 aryl group unsubstituted or substituted with a halogen atom; a C2-C40 heteroaryl group unsubstituted or substituted with a halogen atom; a C7-C15 alkylaryl group unsubstituted or substituted with a halogen atom; a C7-C15 trialkylsilyl group unsubstituted or substituted with a halogen atom; or a C7-C15 aralkyl group unsubstituted or substituted with a halogen atom, and

k may be an integer of 1 to 20.

For example, the halogen substituted in an alkyl group, an alkenyl group, an alkynyl group, a cycloalkyl group, an aryl group, a heteroaryl group, an alkylaryl group, a trialkylsilyl group, or an aralkyl group included in the polar group containing the heteroatom may be fluorine (F).

For example, the compound represented by Formula 1 may be represented by Formula 1-1 below, and the compound represented by Formula 2 may be represented by Formula 2-1 below.

Formula 1-1

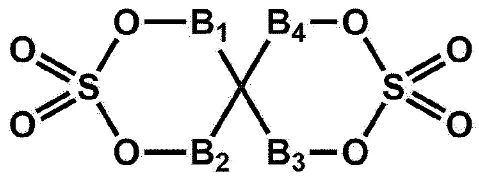

Formula 2-1

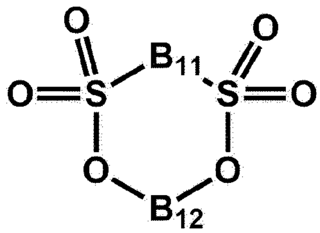

In Formula 1-1 and Formula 2-1,

$B_1$ to $B_4$, $B_{11}$, and $B_{12}$ may be each independently $-C(E_1)(E_2)-$; a carbonyl group; or a sulfinyl group,

$E_1$ and $E_2$ may be each independently hydrogen; a halogen atom; a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C20 cycloalkenyl group unsubstituted or substituted with a halogen atom; a C3-C20 heterocyclyl group unsubstituted or substituted with a halogen atom; a C6-C40 aryl group unsubstituted or substituted with a halogen atom; or a C2-C40 heteroaryl group unsubstituted or substituted with a halogen atom.

For example, $E_1$ and $E_2$ may be each independently hydrogen; a halogen atom; a C1-C10 alkyl group unsubstituted or substituted with a halogen atom; a C6-C40 aryl group unsubstituted or substituted with a halogen atom; or a C2-C40 heteroaryl group unsubstituted or substituted with a halogen atom.

For example, $E_1$ and $E_2$ may be each independently selected from among hydrogen, F, Cl, Br, I, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

For example, $E_1$ and $E_2$ may be each independently hydrogen, fluorine (F), a methyl group, an ethyl group, a trifluoromethyl group, a tetrafluoroethyl group, or a phenyl group.

For example, the compound represented by Formula 1 may be represented by Formula 1-2 below, and the compound represented by Formula 2 may be represented by Formula 2-2 below.

Formula 1-2

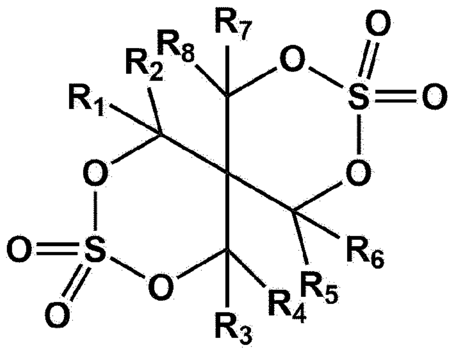

Formula 2-2

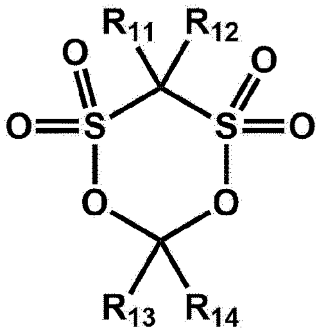

In Formula 1-2 and Formula 2-2,
$R_1$ to $R_8$ and $R_{11}$ to $R_{14}$ may be each independently hydrogen; a halogen atom; a C1-C20 alkyl group unsubstituted or substituted with a halogen atom; a C6-C40 aryl group unsubstituted or substituted with a halogen atom; or a C2-C40 heteroaryl group unsubstituted or substituted with a halogen atom.

For example, $R_1$ to $R_8$ and $R_{11}$ to $R_{14}$ may be each independently selected from among hydrogen, F, Cl, Br, I, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

For example, $R_1$ to $R_8$ and $R_{11}$ to $R_{14}$ may be each independently hydrogen, F, a methyl group, an ethyl group, a propyl group, a trifluoromethyl group, a tetrafluoroethyl group, or a phenyl group.

In particular, the compound represented by Formula 1 may be represented by one selected from Formulas 1-3 to 1-11.

Formula 1-3

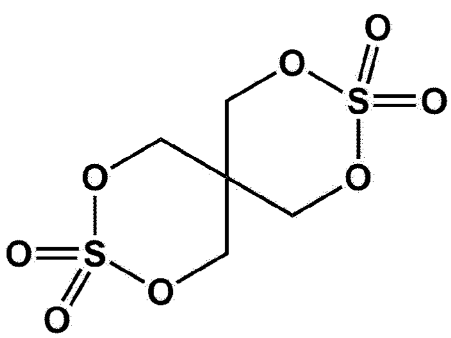

Formula 1-4

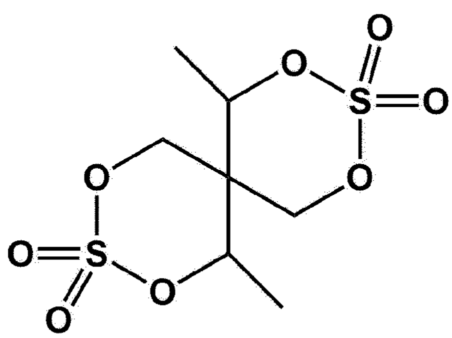

Formula 1-5

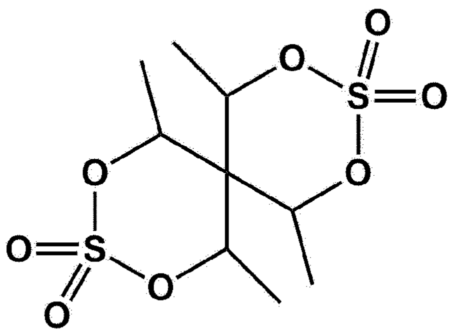

Formula 1-6

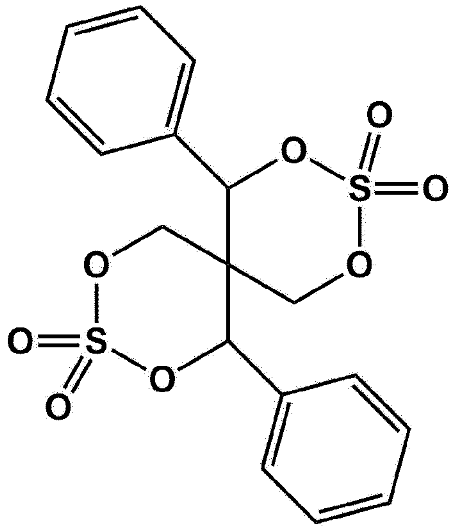

Formula 1-7

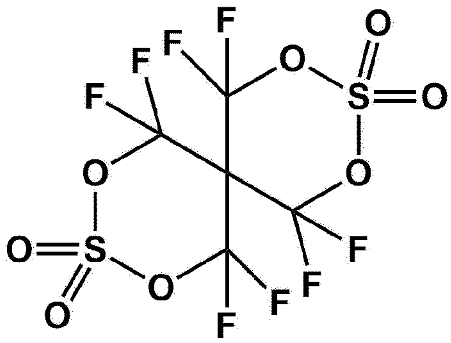

Formula 1-8

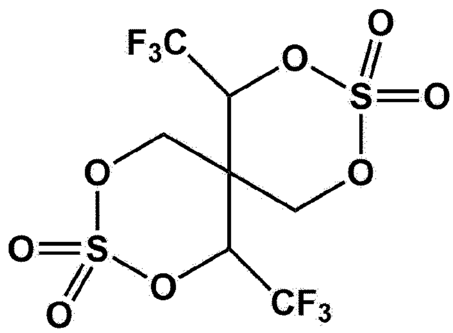

*87 Formula 1-9

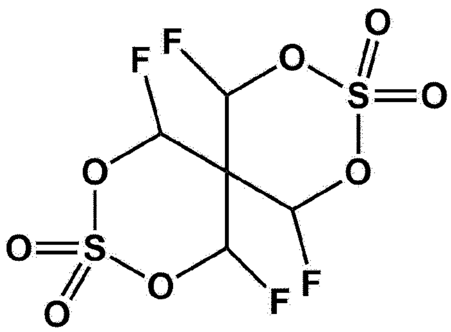

Formula 1-10

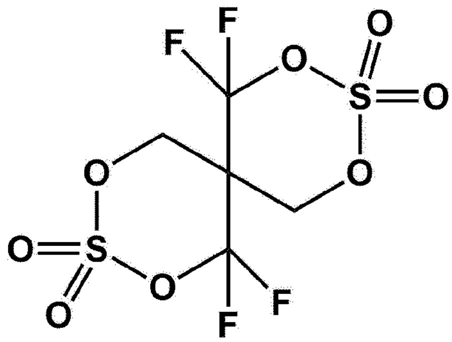

Formula 1-11

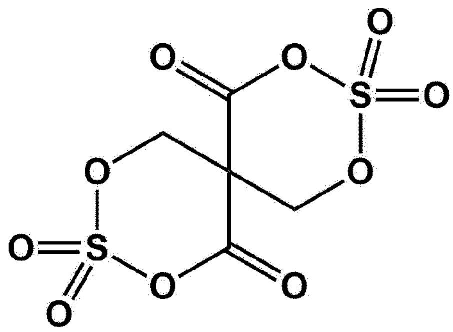

In particular, the compound represented by Formula 2 may be represented by one selected from among Formulas 2-3 to 2-12.

Formula 2-3

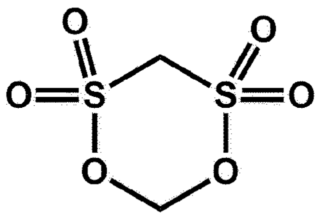

Formula 2-4

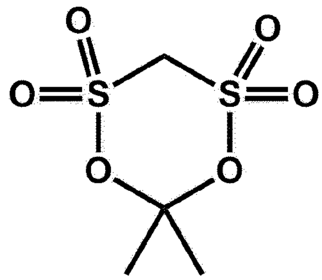

Formula 2-5

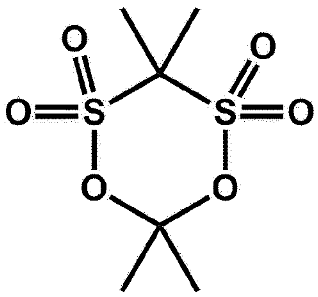

Formula 2-6

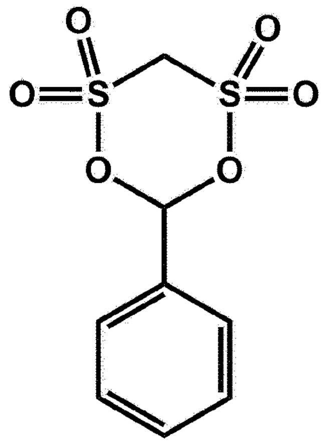

Formula 2-7

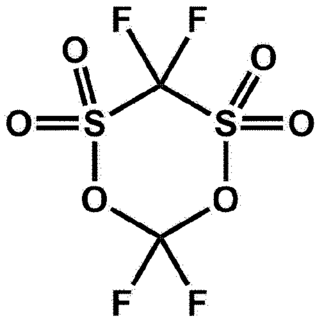

Formula 2-8

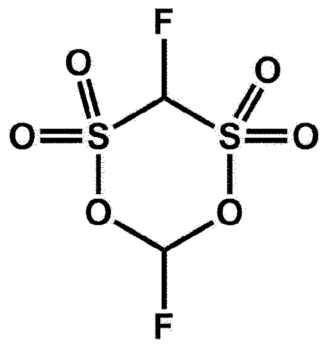

Formula 2-9

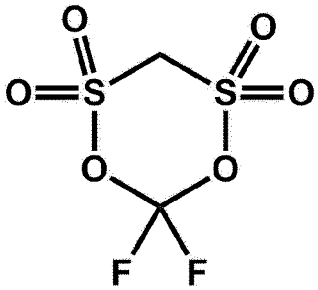

Formula 2-10

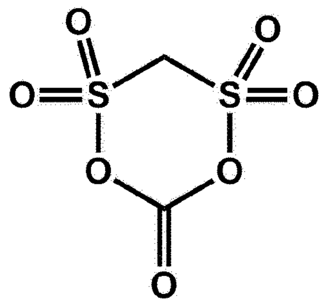

Formula 2-11

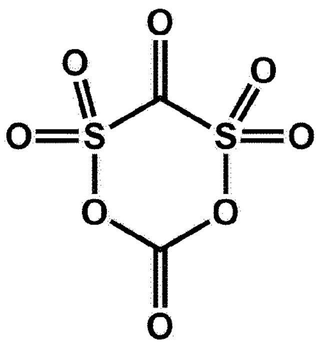

Formula 2-12

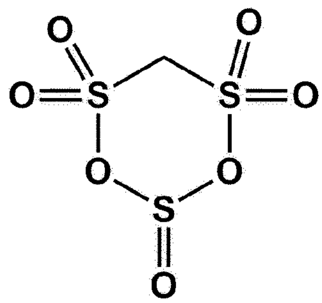

As used herein, a and b in "$C_a$-$C_b$" represent the number of carbon atoms in a functional group. Here, the functional group may include a to b number of carbon atoms. For example, "$C_1$-$C_4$ alkyl group" refers to an alkyl group having 1 to 4 carbon atoms, such as $CH_3$-, $CH_3CH_2$-, $CH_3CH_2CH_2$-, $(CH_3)_2CH$-, $CH_3CH_2CH_2CH_2$-, $CH_3CH_2CH(CH_3)$-, and $(CH_3)_3C$-.

A particular radical may be called a mono-radical or a di-radical depending on the context. For example, when a substituent group needs two binding sites for binding with the rest of the molecule, the substituent may be understood as a di-radical. For example, a substituent group specified as an alkyl group that needs two binding sites may be a di-radical, such as -$CH_2$-, -$CH_2CH_2$-, and -$CH_2CH(CH_3)CH_2$-. The term "alkylene" as used herein clearly indicates that the radical is a di-radical.

The terms "alkyl group" and "alkylene group" as used herein refer to a branched or unbranched aliphatic hydrocarbon group. In an embodiment, the alkyl group may be substituted or unsubstituted. Examples of the alkyl group include, without being limited to a methyl group, an ethyl group, a propyl group, an isopropyl group, a butyl group, an isobutyl group, a tert-butyl group, a pentyl group, a hexyl group, a cyclopropyl group, a cyclopentyl group, a cyclohexyl group, and a cycloheptyl group, each of which may be optionally substituted or unsubstituted. In an embodiment, the alkyl group may have 1 to 6 carbon atoms. For example, a $C_1$-$C_6$ alkyl group may be methyl, ethyl, propyl, isopropyl, butyl, iso-butyl, sec-butyl, pentyl, 3-pentyl, hexyl, or the like, without being necessarily limited thereto.

The term "cycloalkyl group" as used herein means a fully saturated carbocyclic ring or ring system. For example, the cycloalkyl group may be cyclopropyl, cyclobutyl, cyclopentyl, and cyclohexyl.

The term "alkenyl group" as used herein refers to a hydrocarbon group having 2 to 20 carbon atoms with at least one carbon-carbon double bond. Examples of the alkenyl group include an ethenyl group, a 1-propenyl group, a 2-propenyl group, a 2-methyl-1-propenyl group, a 1-butenyl group, a 2-butenyl group, a cyclopropenyl group, a cyclopentenyl group, a cyclohexenyl group, and a cycloheptenyl group. In an embodiment, the alkenyl group may be substituted or unsubstituted. In an embodiment, the alkenyl group may have 2 to 40 carbon atoms.

The term "alkynyl group" as used herein refers to a hydrocarbon group having 2 to 20 carbon atoms with at least one carbon-carbon triple bond. Examples of the alkynyl group include an ethynyl group, a 1-propynyl group, a 1-butynyl group, and a 2-butynyl group, but are not limited thereto. In an embodiment, the alkynyl group may be substituted or unsubstituted. In an embodiment, the alkynyl group may have 2 to 40 carbon atoms.

The term "aromatic" as used herein refers to a ring or ring system with a conjugated n electron system, and may refer to a carbocyclic aromatic group (e.g., a phenyl group) and a heterocyclic aromatic group (e.g., pyridine). In this regard, an aromatic ring system as a whole may include a monocyclic ring or a fused polycyclic ring (i.e., a ring that shares adjacent atom pairs).

The term "aryl group" as used herein refers to an aromatic ring or ring system (i.e., a ring fused from at least two rings that shares two adjacent carbon atoms) having only carbon atoms in its backbone. When the aryl group is a ring system, each ring in the ring system is aromatic. Examples of the aryl group include a phenyl group, a biphenyl group, a naphthyl group, a phenanthrenyl group, and naphthacenyl group, but are not limited thereto. These aryl groups may be substituted or unsubstituted.

The term "heteroaryl group" as used herein refers to an aromatic ring system with one ring or plural fused rings, in which at least one ring atom is not carbon, i.e., a heteroatom. In the fused ring system, at least one heteroatom may be present in only one ring. For example, the heteroatom may be oxygen, sulfur, or nitrogen, but is not limited thereto. Examples of the heteroaryl group include, but are not limited to a furanyl group, a thienyl group, an imidazolyl group, a quinazolinyl group, a quinolinyl group, an isoquinolinyl group, a quinoxalinyl group, a pyridinyl group, a pyrrolyl group, an oxazolyl group, and an indolyl group.

The terms "aralkyl group" and "alkylaryl group" as used herein refer to an aryl group linked as a substituent via an alkylene group, such as a $C_7$-$C_{14}$ aralkyl group. Examples of the aralkyl group or alkylaryl group include a benzyl group, a 2-phenylethyl group, a 3-phenylpropyl group, and a naphthylalkyl group, but are not limited thereto. In an embodiment, the alkylene group may be a lower alkylene group (i.e., a $C_1$-$C_4$ alkylene group).

The term "cycloalkenyl group" as used herein refers to a non-aromatic carbocyclic ring or ring system with at least one double bond. For example, the cycloalkenyl group may be a cyclohexenyl group.

The term "heterocyclic group" as used herein refers to a non-aromatic ring or ring system having at least one heteroatom in its ring backbone.

The term "halogen" as used herein refers to a stable element belonging to Group 17 of the periodic table, for example, fluorine, chlorine, bromine, or iodine. In particular, the halogen may be fluorine and/or chlorine.

In the present specification, a substituent may be derived by substitution of at least one hydrogen atom in an unsubstituted mother group with another atom or a functional group. Unless stated otherwise, the term "substituted" means that the aforementioned functional groups are substituted with at least one substituent selected from a $C_1$-$C_{40}$ alkyl group, a $C_2$-$C_{40}$ alkenyl group, a $C_3$-$C_{40}$ cycloalkyl group, a $C_3$-$C_{40}$ cycloalkenyl group, and a $C_7$-$C_{40}$ aryl group. The phrase "optionally substituted" as used herein means that the aforementioned functional groups may be substituted with the aforementioned substituents.

An organic electrolyte according to another embodiment may include a first lithium salt; an organic solvent; and an additive including the compound represented by Formula 1 above and the compound represented by Formula 2 above.

The content of the compound represented by Formula 1 in the organic electrolyte may be 0.1% to 5.0%, 0.2% to 5.0%, 0.1% to 4.0%, 0.1% to 3.0%, 0.2% to 3.0%, or 0.2% to 2.0% with respect to the total weight of the organic electrolyte. When the above ranges are satisfied, a lithium battery including the organic electrolyte may reduce resistance changes at a high temperature.

The content of the compound represented by Formula 2 in the organic electrolyte may be 0.1% to 5.0%, 0.2% to 5.0%, 0.1% to 4.0%, 0.1% to 3.0%, 0.2% to 3.0%, or 0.2% to 2.0% with respect to the total weight of the organic electrolyte. When the above ranges are satisfied, a lithium battery including the organic electrolyte may reduce the amount of gas generation at a high temperature.

The ratio of the content of the compound represented by Formula 2 to the content of the compound represented by Formula 1 in the organic electrolyte may be 1:10 to 10:1, 2:10to 10:1, 2.5:10 to 10:1 ,5:10 to 10:1, 1:10 to 10:2, 1:10 to 10:2.5, 1:10 to 10:5, or 2:10 to 10:5.

The lithium salt used in the organic electrolyte is not particularly limited, and may be any lithium salt available in the art. For example, the lithium salt may include one or more selected from among $LiPF_6$, $LiBF_4$, $LiSbF_6$, $LiAsF_6$,

$LiClO_4$, LiCFsSOs, $Li(CF_3SO_2)_2N$, $LiC_4F_9SO_3$, $LiAlO_2$, $LiAlCl_4$, $LiN(C_xF_{2x+1}SO_2)(C_yF_{2y+1}SO_2)$ ($2 \leq x \leq 20$, $2 \leq y \leq 20$), LiCl, and LiI.

In the organic electrolyte, the concentration of the lithium salt may be 0.01 M to 2.0 M, but is not necessarily limited to the aforementioned range and may be adjusted to an appropriate concentration as necessary. Within the above concentration ranges, further improved battery characteristics may be obtained.

The organic solvent in the organic electrolyte may include a low-boiling point solvent. The low-boiling point solvent refers to a solvent having a boiling point of 200 °C or less, at 25 °C and 1 atm.

For example, the organic solvent may include at least one selected from among dialkyl carbonates, cyclic carbonates, linear or cyclic esters, linear or cyclic amides, aliphatic nitriles, linear or cyclic ethers, and derivatives thereof.

More specifically, the organic solvent may include at least one selected from among dimethyl carbonate (DMC), ethyl methyl carbonate (EMC), methyl propyl carbonate, ethyl propyl carbonate, diethyl carbonate (DEC), dipropyl carbonate, propylene carbonate (PC), ethylene carbonate (EC), fluoroethylene carbonate (FEC)), butylene carbonate, ethyl propionate, ethyl butyrate, acetonitrile, succinonitrile (SN), dimethyl sulfoxide, dimethylformamide, dimethylacetamide, gamma-valerolactone, gamma-butyrolactone and tetrahydrofuran. However, the organic solvent is not necessarily limited to the aforementioned components and may be any low-boiling point solvent available in the art.

The organic electrolyte may be in a liquid state or a gel state. The organic electrolyte may be prepared by adding a lithium salt and the above-described additive to the above-described organic solvent.

In addition, the organic electrolyte may further include other additives in addition to the compound represented by Formula 1 and the compound represented by Formula 2. By additional inclusion of other additives, the lithium battery may have further improved performance.

The other additives that may be further included in the organic electrolyte may be a cyclic carbonate compound, a second lithium salt, and the like.

For example, the organic electrolyte may further include a cyclic carbonate compound as an additive. The cyclic carbonate compound for use as an additive may be selected from among vinylene carbonate (VC); a vinylene carbonate substituted with one or more substituents selected from among a halogen atom, a cyano group (CN) and a nitro group ($NO_2$); vinyl ethylene carbonate (VEC); a vinyl ethylene carbonate substituted with one or more substituents selected from among a halogen atom, a cyano group (CN) and a nitro group ($NO_2$); fluoroethylene carbonate (FEC); and a fluoroethylene carbonate substituted with one or more substituents selected from among a halogen atom, a cyano group (CN) and a nitro group ($NO_2$). As the organic electrolyte further includes a cyclic carbonate compound as an additive, charge-discharge characteristics of a lithium battery including the organic electrolyte may be further improved.

In the organic electrolyte, the content of a cyclic carbonate-based compound may be 0.01 wt% to 10 wt% with respect to the total weight of the organic electrolyte, but without being limited to the aforementioned range, the cyclic carbonate-based compound may be used in an appropriate amount as necessary. For example, the content of the cyclic carbonate-based compound in the organic electrolyte may be 0.1 wt% to 10 wt%, 1 wt% to 10 wt%, 3 wt% to 10 wt%, 1 wt% to 7 wt%, or 3 wt% to 7 wt% with respect to the total weight of the organic electrolyte. Within the above ranges, further improved battery characteristics may be obtained.

For example, the organic electrolyte may further include a second lithium salt as an additive. The second lithium salt may be distinguished from the first lithium salt, and an anion of the second lithium salt may be oxalate, $PO_2F_2$-, $N(SO_2F)_2$-, or the like. For example, the second lithium salt may be a compound represented by Formulas 3 to 10.

Formula 3

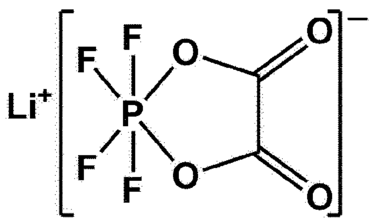

Formula 4

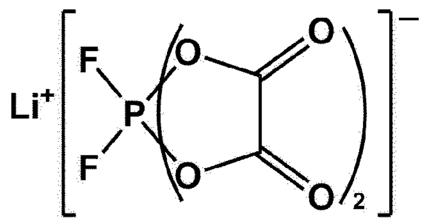

Formula 5

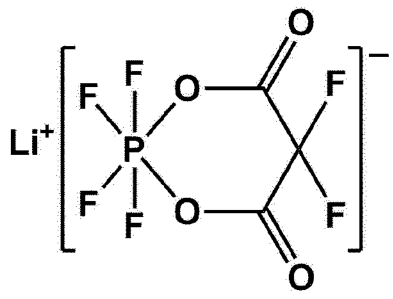

Formula 6

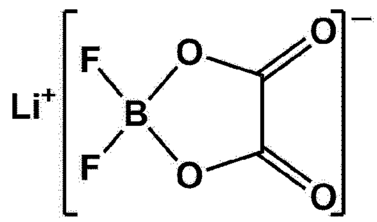

Formula 7

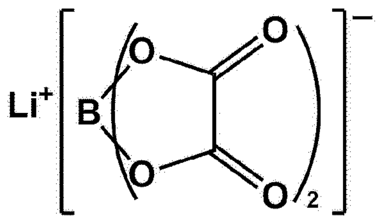

Formula 8

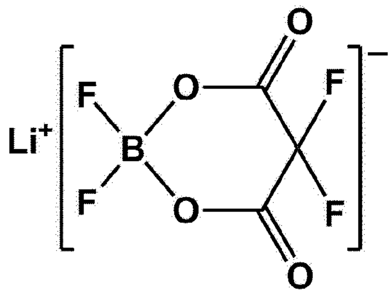

Formula 9

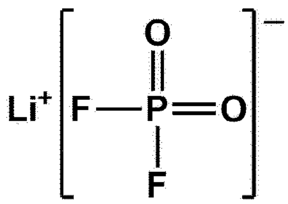

Formula 10

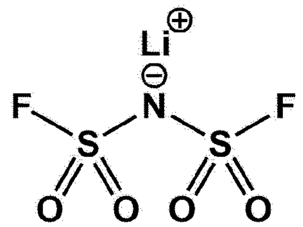

In the organic electrolyte, the content of the second lithium salt may be 0.1 wt% to 5 wt% with respect to the total weight of the organic electrolyte, but without being limited to the aforementioned range, the second lithium salt may be used in an appropriate amount as necessary. For example, the content of the second lithium salt in an organic electrolyte may be 0.1 wt% to 5 wt% with respect to the total weight of the organic electrolyte. For example, the content of the second lithium salt in the organic electrolyte may be 0.1 wt% to 4 wt% with respect to the total weight of the organic electrolyte. For example, the content of the second lithium salt in the organic electrolyte may be 0.1 wt% to 3 wt% with respect to the total weight of the organic electrolyte. For example, the content of the second lithium salt in the organic electrolyte may be 0.1 wt% to 2 wt% with respect to the total weight of the organic electrolyte. For example, the content of the second lithium salt in the organic electrolyte may be 0.2 wt% to 2 wt% with respect to the total weight of the organic electrolyte. For example, the content of the second lithium salt in the organic electrolyte may be 0.2 wt% to 1.5 wt% with respect to the total weight of the organic electrolyte. Within the above ranges, further improved battery characteristics may be obtained.

The organic electrolyte may be in a liquid state or a gel state. The organic electrolyte may be prepared by adding the first lithium salt and the above-described additive to the above-described organic solvent.

*141A lithium battery according to another embodiment may include a positive electrode; a negative electrode; and the organic electrolyte described above. The lithium battery is not limited to any particular type and may include lithium primary batteries as well as lithium secondary batteries, such as a lithium ion battery, a lithium ion polymer battery, a lithium sulfur battery, and the like.

For example, the negative electrode in the lithium battery may include graphite. For example, the positive electrode in the lithium battery may include a nickel-containing layered-structure lithium transition metal oxide. For example, a lithium battery may have a high voltage of 3.80 V or more. For example, a lithium battery may have a high voltage of 4.0 V or more. For example, a lithium battery may have a high voltage of 4.35 V or more.

For example, a lithium battery may be prepared as follows.

First, a positive electrode may be prepared.

For example, a positive electrode active material composition containing a mixture of a positive electrode active material, a conductive material, a binder, and a solvent may be prepared. The positive electrode active material composition may be directly coated on a metal current collector to thereby form a positive electrode plate. Alternatively, the positive electrode active material composition may be cast on a separate support, and a film exfoliated from the support may be laminated on a metal current collector to thereby form a positive electrode plate. The positive electrode is not limited to the aforementioned types and may be a type other than the aforementioned types.

For the positive electrode active material, any lithium-containing metal oxide commonly available in the art may be used without limitations. For example, the positive electrode active material may be at least one of composite oxides of lithium with a metal selected from among cobalt, manganese, nickel, and a combination thereof. Specifically, the positive electrode active material may use a compound represented by any one of the following formulas: $Li_aA_{1-b}B_bD_2$ (in the formula, $0.90 \le a \le 1.8$, and $0 \le b \le 0.5$); $Li_aE_{1-b}B_bO_{2-c}D_c$ (in the formula, $0.90 \le a \le 1.8$, $0 \le b \le 0.5$, and $0 \le c \le 0.05$); $LiE_{2-b}B_bO_{4-c}D_c$ (in the formula, $0 \le b \le 0.5$, and $0 \le c \le 0.05$); $Li_aNi_{1-b-c}Co_bB_cD_\alpha$ (in the formula, $0.90 \le a \le 1.8$, $0 \le b \le 0.5$, $0 \le c \le 0.05$, and $0 < \alpha \le 2$); $Li_aNi_{1-b-c}Co_bB_cO_{2-\alpha}F_\alpha$ (in the formula, $0.90 \le a \le 1.8$, $0 \le b \le 0.5$, $0 \le c \le 0.05$, and $0 < \alpha < 2$); $Li_aNi_{1-b-c}Co_bB_cO_{2-\alpha}F_2$ (in the formula, $0.90 \le a \le 1.8$, $0 \le b \le 0.5$, $0 \le c \le 0.05$, and $0 < \alpha < 2$); $Li_aNi_{1-b-c}Mn_bB_cD_\alpha$ (in the formula, $0.90 \le a \le 1.8$, $0 \le b \le 0.5$, $0 \le c \le 0.05$, and $0 < \alpha \le 2$); $Li_aNi_{1-b-c}Mn_bB_cO_{2-\alpha}F_\alpha$ (in the formula, $0.90 \le a \le 1.8$, $0 \le b \le 0.5$, $0 \le c \le 0.05$, and $0 < \alpha < 2$); $Li_aNi_{1-b-c}Mn_bB_cO_{2-\alpha}F_2$ (in the formula, $0.90 \le a \le 1.8$, $0 \le b \le 0.5$, $0 \le c \le 0.05$, and $0 < \alpha < 2$); $Li_aNi_bE_cG_dO_2$ (in the formula, $0.90 \le a \le 1.8$, $0 \le b \le 0.9$, $0 \le c \le 0.5$, and $0.001 \le d \le 0.1$); $Li_aNi_bCo_cMn_dGeO_2$ (in the formula, $0.90 \le a \le 1.8$, $0 \le b \le 0.9$, $0 \le c \le 0.5$, $0 \le d \le 0.5$, and $0.001 \le e \le 0.1$); $Li_aNiG_bO_2$ (in the formula, $0.90 \le a \le 1.8$, and $0.001 \le b \le 0.1$); $Li_aCoG_bO_2$ (in the formula, $0.90 \le a \le 1.8$, and $0.001 \le b \le 0.1$); $Li_aMnG_bO_2$ (in the formula, $0.90 \le a \le 1.8$, and $0.001 \le b \le 0.1$); $Li_aMn_2G_bO_4$ (in the formula, $0.90 \le a \le 1.8$, and $0.001 \le b \le 0.1$); $QO_2$; $QS_2$; $LiQS_2$; $V_2O_5$; $LiV_2O_5$; $LiIO_2$; $LiNiVO_4$; $Li_{(3-f)}J_2(PO_4)_3$ ($0 \le f \le 2$); $Li_{(3-f)}Fe_2(PO_4)_3$ ($0 \le f \le 2$); and $LiFePO_4$.

In the above formulas, A may be Ni, Co, Mn, or a combination thereof; B may be Al, Ni, Co, Mn, Cr, Fe, Mg, Sr, V, an rare-earth element, or a combination thereof; D may be O, F, S, P, a combination thereof; E may be Co, Mn, a combination thereof; F may be F, S, P, a combination thereof; G may be Al, Cr, Mn, Fe, Mg, La, Ce, Sr, V, or a combination thereof; Q may be Ti, Mo, Mn, or a combination thereof; I may be Cr, V, Fe, Sc, Y, or a combination thereof; and J may be V, Cr, Mn, Co, Ni, Cu, or a combination thereof.

The positive electrode active material may be, for example, a lithium transition metal oxide represented by Formulas 11 to 14 below.

Formula 11 $Li_aNi_xCo_yM_zO_{2-b}A_b$

In Formula 11,

$1.0 \le a \le 1.2$, $0 \le b \le 0.2$, $0.6 \le x < 1$, $0 < y \le 0.3$, $0 < z \le 0.3$, and $x+y+z=1$ may be satisfied, and
M may be at least one selected from among manganese (Mn), vanadium (V), magnesium (Mg), gallium (Ga), silicon (Si), tungsten (W), molybdenum (Mo), iron (Fe), chromium (Cr)), copper (Cu), zinc (Zn), titanium (Ti), aluminum (Al), and boron (B), and A may be F, S, Cl, Br, or a combination thereof.

Formula 12 $LiNi_xCo_yMn_zO_2$

Formula 13 $LiNi_xCo_yAl_zO_2$

In Formulas 12 and 13, $0.6 \le x \le 0.95$, $0 < y \le 0.2$, $0 < z \le 0.2$, and $x+y+z=1$ may be satisfied.

Formula 14 $LiNi_xCo_yMn_xAl_wO_2$

In Formula 14, $0.6 \le x \le 0.95$, $0 < y \le 0.2$, $0 < z \le 0.2$, $0 < w \le 0.2$, and $x+y+z+w=1$ may be satisfied.

For example, the positive electrode active material may be $LiCoO_2$, $LiMn_xO_{2x}$ (x=1, 2), $LiNi_{1-x}Mn_xO_{2x}$ (0<x<1), $LiNi_{1-x-y}Co_xMn_yO_2$ ($0 \le x \le 0.5$, $0 \le y \le 0.5$), $LiNi_{1-x-y}Co_xAl_yO_2$ ($0 \le x \le 0.5$, $0 \le y \le 0.5$), $LiFePO_4$ or the like. Needless to say, a lithium-containing metal oxide having a coating layer on a surface thereof may be used as the positive electrode active material, or a mixture of a lithium-containing metal oxide and a compound having a coating layer on the surface of a lithium-containing metal oxide may be used. This coating layer may include a coating element compound, such as an oxide and a hydroxide of a coating element, oxyhydroxide of a coating element, an oxycarbonate of a coating element, and a hydroxycarbonate of a coating element. A compound forming these coating layers may be amorphous or crystalline.

The coating element included in the coating layer may include Mg, Al, Co, K, Na, Ca, Si, Ti, V, Sn, Ge, Ga, B, As, Zr, or a mixture thereof. For the process of forming the coating layer, any coating method that is capable of coating the above compound by using such elements, without having an adverse effect on physical properties of positive electrode active material may be used without limitation (e.g., spray coating, precipitation, etc.). Since such methods are commonly understood by those skilled in the art, they will not be described in further detail.

Examples of the conductive material may include carbon black, graphite powder, and the like. However, the conductive material is not limited to the aforementioned components and may be any conductive material available in the art.

For the binder, a vinylidene fluoride/hexafluoropropylene copolymer, polyvinylidene fluoride (PVDF), polyacrylonitrile, polymethyl methacrylate, polytetrafluoroethylene, and a mixture thereof, or a styrene butadiene rubber-based polymer, etc. may be used. However, the binder is not limited to the aforementioned components and may be any binder available in the art.

For the solvent, N-methyl pyrrolidone, acetone, or water, etc. may be used, but the solvent is not limited thereto and may be any solvent that is usable in the art.

The respective amounts of the positive electrode active material, the conductive material, the binder, and the solvent are at a level commonly used in lithium batteries. Depending on the use and configuration of a lithium battery, one or more of the conductive material, the binder, and the solvent may be omitted.

Next, a negative electrode may be prepared.

For example, a negative electrode active material composition may be prepared by combining a negative electrode active material, a conductive material, a binder, and a solvent. The negative electrode active material composition may be directly coated and dried on a metal current collector to thereby form a negative electrode plate. Alternatively, the negative electrode active material composition may be cast on a separate support, and a film exfoliated from the support may be laminated on a metal current collector to thereby form a negative electrode plate.

The negative electrode active material may be any material available in the art as a negative electrode active material of a lithium battery. For example, the negative electrode active material may include one or more selected from among lithium metal, a metal alloyable with lithium, a transition metal oxide, a non-transition metal oxide, and a carbon-based material.

Examples of the metal alloyable with lithium may include Si, Sn, Al, Ge, Pb, Bi, Sb, a Si-Y alloy (wherein Y is an alkali metal, an alkaline earth metal, an element in Group 13, an element in Group 14, a transition metal, a rare-earth metal, or a combination thereof, but not Si), a Sn-Y alloy (wherein Y is an alkali metal, an alkaline earth metal, an element in Group 13, an element in Group 14, a transition metal, a rare-earth metal, or a combination thereof, but not Sn), and the like. Y may be Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ti, Ge, P, As, Sb, Bi, S, Se, Te, Po, or a combination thereof.

For example, the transition metal oxide may be a lithium titanium oxide, a vanadium oxide, a lithium vanadium oxide, and the like.

For example, the non-transition metal oxide may be $SnO_2$, $SiO_x$ ($0<x<2$), and the like.

For example, the carbon-based material may be a crystalline carbon, an amorphous carbon, or a mixture thereof. Examples of the crystalline carbon may include graphite, including artificial graphite or natural graphite in shapeless, plate, flake, spherical or fiber form, and examples of the amorphous carbon may include soft carbon (low-temperature calcined carbon) or hard carbon, mesophase pitch carbides, calcined cokes, and the like.

The negative electrode active material composition may utilize the same conductive material and binder as in the positive electrode active material composition above.

The respective amounts of the negative electrode active material, the conductive material, the binder, and the solvent are at a level commonly used in lithium batteries. Depending on the use and configuration of a lithium battery, one or more of the conductive material, the binder, and the solvent may be omitted.

Next, a separator to be positioned between the positive electrode and the negative electrode may be prepared.

The separator may be any separator commonly used in a lithium battery. Any separator capable of retaining a large quantity of electrolyte solution while exhibiting low resistance to ion migration in the electrolyte may be used. For example, the separator may be any material selected from among glass fiber, polyester, Teflon, polyethylene, polypropylene, polytetrafluoroethylene (PTFE), and a combination thereof. In addition, the separator may be in the form of nonwoven fabric or woven fabric. For example, a lithium ion battery may include a rollable separator formed of polyethylene, polypropylene, or the like, and a lithium ion polymer battery may include a separator which has an excellent impregnation ability with respect to an organic electrolyte. For example, the separator may be prepared as follows.

A separator composition may be prepared by mixing a polymer resin, a filler, and a solvent. The separator composition may be directly coated and dried on top of an electrode to thereby form a separator. Alternatively, the separator composition may be cast and dried on a support, and a separator film exfoliated from the support may be laminated on top of an electrode to thereby form a separator.

The polymer resin used in the separator preparation is not limited to any particular type and may be any material

available for use in a binding material of an electrode plate. For example, a vinylidene fluoride/hexafluoropropylene copolymer, polyvinylidene fluoride (PVDF), polyacrylonitrile, polymethyl methacrylate, or a mixture thereof may be used as the polymer resin.

Next, the above-described organic electrolyte may be prepared.

As shown in FIG. 1, a lithium battery 1 may include a positive electrode 3, a negative electrode 2, and a separator 4. The positive electrode 3, the negative electrode 2, and the separator 4 may be wound or folded so as to be accommodated in a battery case 5. Then, the battery case 5 may be injected with an organic electrolyte and sealed with a cap assembly 6, thereby completing the preparation of the lithium battery 1. The battery case may be a cylindrical type, a rectangular type, a thin-film type, and the like.

A separator may be arranged between a positive electrode and a negative electrode, thereby forming a battery structure. The battery structure may be stacked in a bi-cell structure and impregnated with an organic electrolyte, and the product obtained therefrom may be accommodated and sealed in a pouch, thereby completing the preparation of a lithium battery.

A plurality of battery structures may be stacked to form a battery pack, and such a battery pack may be used in any and all devices that require high capacity and high power output. For example, such a battery pack may be used in a laptop computer, a smartphone, an electric vehicle, and the like.

In addition, the lithium battery due to having excellent lifespan characteristics and high-rate characteristics, may be used in an electric vehicle (EV). For example, the lithium battery may be used in a hybrid vehicle, such as a plug-in hybrid electric vehicle (PHEV), and the like. Also, the lithium battery may be used in any field that requires a large amount of energy storage. For example, the lithium battery may be used in an electric bicycle, a power tool, and the like.

Exemplary embodiments will be described in greater detail through Examples and Comparative Examples below. However, it will be understood that Examples are provided only to illustrate the present inventive concept and should not be construed as limiting the scope of the present inventive concept.

(Preparation of Organic Electrolyte)

Preparation Example 1: Formula 1-3 1 wt%, Formula 2-3 0.5 wt%, and FEC 5 wt%

1.0 M $LiPF_6$ as a lithium salt, 1 wt% of the compound represented by Formula 1-3 below, 0.5 wt% of the compound (MMDS) represented by Formula 2-3 below, and 5 wt% of fluoroethylene carbonate (FEC) were added to a mixed solvent containing ethylene carbonate (EC), ethyl methyl carbonate (EMC) and dimethyl carbonate (DMC) in a volume ratio of 2:2:6 to thereby complete the preparation of an organic electrolyte.

Formula 1-3

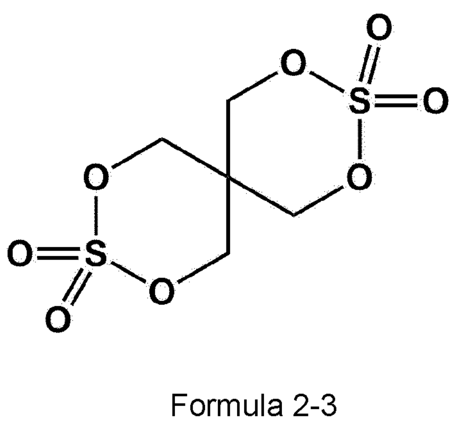

Formula 2-3

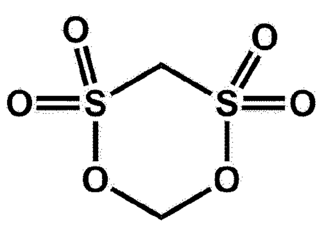

Preparation Examples 2 to 5 and Comparative Preparation Examples 1 to 4

An organic electrolyte was prepared following the same process as Preparation Example 1 except that the types and amounts of additives used are as shown in Table 1.

[Table 1]

| | Additive (Formula 1) | | Additive (Formula 2) | | FEC (wt%) |
|---|---|---|---|---|---|
| | Type | wt% | Type | wt% | |
| Preparation Example 1 | Formula 1-3 | 1 | Formula 2-3 | 0.5 | 5 |
| Preparation Example 2 | Formula 1-3 | 2 | Formula 2-3 | 0.5 | 5 |
| Preparation Example 3 | Formula 1-3 | 5 | Formula 2-3 | 0.5 | 5 |
| Preparation Example 4 | Formula 1-3 | 1 | Formula 2-3 | 2 | 5 |
| Preparation Example 5 | Formula 1-3 | 1 | Formula 2-3 | 5 | 5 |
| Comparative Preparation Example 1 | Formula 1-3 | 1 | Formula 2-3 | - | 5 |
| Comparative Preparation Example 2 | Formula 1-3 | - | Formula 2-3 | 0.5 | 5 |
| Comparative Preparation Example 3 | Formula 1-3 | - | Formula 2-3 | - | 5 |
| Comparative Preparation Example 4 | Formula 1-12 | 1 | Formula 2-3 | 0.5 | 5 |

Formula 1-12

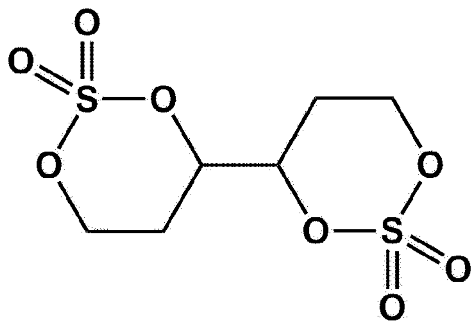

(Preparation of Lithium Battery)

Example 1

(Preparation of Negative Electrode)

98 wt% of artificial graphite, 1.0 wt% of a styrene-butadiene rubber (SBR) binder, and 1.0 wt% of carboxymethylcellulose (CMC) were mixed together, and the resulting mixture was added to distilled water and stirred for 60 minutes using a mechanical stirrer, to thereby prepare a negative electrode active material slurry. The slurry was applied, using a doctor blade, onto a 10 μm-thick copper current collector to a thickness of about 60 μm, and the coated current collector was dried in a hot-air dryer at 100 °C for 0.5 hours, followed by further drying in vacuum at 120 °C for 4 hours, and roll-pressed, thereby completing the preparation of a negative electrode plate.

(Preparation of Positive Electrode)

97.45 wt% of $LiNi_{0.88}Co_{0.09}Al_{0.03}O_2$, 0.5 wt% of artificial graphite powder as a conductive material, 0.7 wt% of carbon black, 0.25 wt% of a modified acrylonitrile rubber, and 1.1 wt% of polyvinylidene fluoride (PVdF) were mixed together, and the resulting mixture was added to N-methyl-2-pyrrolidone solvent and stirred for 30 minutes using a mechanical stirrer, to thereby prepare a positive electrode active material slurry. The slurry was applied, using a doctor blade, onto a 20 μm-thick aluminum current collector to a thickness of about 60 μm, and the coated current collector

was dried in a hot-air dryer at 100 °C for 0.5 hours, followed by further drying in vacuum at 120 °C for 4 hours, and roll-pressed, thereby completing the preparation of a positive electrode plate.

A lithium battery was prepared using a 14 μm-thick polyethylene separator, a positive electrode side of which was coated with ceramic, as a separator, and the organic electrolyte prepared in Example 1 as an electrolyte.

Examples 2 and 3 and Comparative Examples 1 to 3

A lithium battery was prepared following the same process as Example 1, except that instead of the organic electrolyte prepared in Preparation Example 1, the respective organic electrolytes prepared in Preparation Examples 2 and 3, and Comparative Examples 1 to 3 were used.

Evaluation Example 1: Evaluation of Room-Temperature (25°C) Charge/Discharge Characteristics

The lithium batteries prepared in Examples 1 to 5 and Comparative Examples 1 to 4 were each charged at a constant current of 0.3 C rate at 25 °C until the voltage reached 4.2 V (vs. Li) and then, the charging was cut-off at a current of 0.05 C rate while maintaining a constant voltage mode at 4.2 V. Subsequently, each battery was discharged at a constant current of 1.0 C rate until the voltage reached 2.5 V (vs. Li) during discharge. This cycle was repeated up to 300 cycles. Throughout the charge-discharge cycles above, a rest period of 10 minutes was provided after each charge/discharge cycle.

A part of the charge-discharge test results is shown in Table 2 below. Capacity retention ratio at the 300th cycle is defined by Equation 1 below.

Equation 1

$$\text{Capacity retention ratio} = [\text{Discharge capacity at } 300^{th} \text{ cycle / Discharge capacity at } 1^{st} \text{ cycle}] \times 100$$

Evaluation Example 2: Evaluation on High-Temperature Stability at 60 °C

The lithium batteries prepared in Examples 1 to 5 and Comparative Examples 1 to 4 were each, at room temperature (25 °C), charged in the 1st cycle with a constant current of 0.3 C rate until the voltage reached 4.2 V, and subsequently charged at a constant voltage at 4.2 V until the current reached 0.05 C, and then discharged at a constant current of 1.0 C rate until the voltage reached 2.5 V.

In the 2nd cycle, each battery was charged at a constant current of 0.3 C rate until the voltage reached 4.2 V, subsequently charged at a constant voltage at 4.2 V until the current reached 0.05 C, and then discharged at a constant current of 1.0 C rate until the voltage reached 2.5 V.

In the 3rd cycle, each battery was charged with a constant current of 0.5 C rate until the voltage reached 4.2 V, subsequently charged at a constant voltage at 4.2 V until the current reached 0.02 C, and then discharged at a constant current of 0.2 C rate until the voltage reached 2.5 V. The discharge capacity at the 3rd cycle was used as standard capacity.

In the 4th cycle, each battery was charged at 0.5 C until the voltage reached 4.2 V, and then charged at a constant voltage at 4.2 V until the current reached 0.02 C. Thereafter, each charged battery was stored in an oven at 60 °C for 30 days and then taken out of the oven, and the discharging of the 4th cycle was performed at 0.2 C rate until the voltage reached 2.5 V.

Equation 2

$$\text{Capacity retention ratio after high-temperature storage [\%]} = [\text{Discharge capacity after high-temperature storage at } 4^{th} \text{ cycle / Reference capacity}] \times 100 \text{ (Reference capacity is a discharge capacity at } 3^{rd} \text{ cycle)}$$

Evaluation Example 3: Evaluation on Direct Current Internal Resistance (DC-IR) after High-Temperature Storage at 60 °C

Among the lithium batteries prepared in Examples 1 to 5 and Comparative Examples 1 to 4, at room temperature

(25 °C), the batteries that were not stored in the 60 °C oven, and the batteries that were stored in the 60 °C oven for 30 days were evaluated for DC-IR as follows.

In the 1st cycle, after charging each battery at a current of 0.5 C until the voltage reached 4.2 V, the charging was cut-off at 0.02 C, followed by 10 minutes of rest. Then, each battery was discharged at 4.0 A for 10 seconds, and without resting, discharged at a constant current of 1.0 A for 10 seconds, and then without resting, discharged again at a constant current of 4.0 A for 4 seconds.

In the series of discharging processes in the 1st cycle, from the ratio of the change in voltage ($\Delta V$) to the change in current ($\Delta I$) between the 8 second point during the discharge at a constant current of 1.0 A and the 3 second point in the second discharge process at a constant current of 4.0 A, DC-IRs ($R= \Delta V/\Delta I$) were calculated and taken as measurement values.

Based on the measured DC-IRs, DC-IR changes calculated from DC-IRs after high-temperature storage are shown in Table 2 below.

Resistance change ratio is represented by Equation 3 below.

Equation 3

Resistance change ratio [%] = [DCIR right after high-temperature storage / Initial DCIR] X 100

Evaluation Example 4: Evaluation on the Amount of Gas Generation after 60 °C High-Temperature Storage

The lithium secondary batteries prepared in Examples 1 to 5 and Comparative Examples 1 to 4 were each measured for the amount of gas generation after storage at 60 °C for 30 days.

While maintaining at 25 °C, the battery was placed in a pressure measurement jig, and after creating a vacuum state inside the jig by using a vacuum pump for 10 minutes, the vacuum valve was closed and a pressure measurement was taken. This measurement value was used as P0. Using a pin inside the pressure measurement jig, an impact was created on the circular cell bottom to release gas inside, and the pressure inside the jig was measured after 10 seconds. This measurement value was used as P1.

The number of moles n of generated gas was calculated by the following equation.

$$n = [(P1 - P0) * Vzig / (R * 298\ K)]$$

Since the volume of 1 mole of gas at 1 atm is about 22,400 mL, the volume of generated gas V can be calculated as n * 22,400 mL. The value thus calculated was taken as the amount of gas generation and is shown in Table 2 below.

[Table 2]

| | Room temperature (25 °C) Capacity retention ratio @300cy (%) | High temperature (60 °C) Capacity retention ratio @30 day (%) | High temperature (60 °C) Resistance change ratio @30 day (%) | High temperature (60 °C) Amount of gas generation @30 day (ml) |
|---|---|---|---|---|
| Example 1 | 87 | 87 | 93 | 0.6 |
| Example 2 | 86 | 86 | 92 | 0.7 |
| Example 3 | 84 | 85 | 95 | 0.7 |
| Example 4 | 85 | 86 | 96 | 0.6 |
| Example 5 | 84 | 85 | 98 | 0.6 |
| Comparative Example 1 | 82 | 85 | 101 | 0.7 |
| Comparative Example 2 | 84 | 83 | 118 | 0.8 |
| Comparative Example 3 | 78 | 82 | 124 | 1.4 |

(continued)

| | Room temperature (25 °C) Capacity retention ratio @300cy (%) | High temperature (60 °C) Capacity retention ratio @30 day (%) | High temperature (60 °C) Resistance change ratio @30 day (%) | High temperature (60 °C) Amount of gas generation @30 day (ml) |
|---|---|---|---|---|
| Comparative Example 4 | 80 | 83 | 110 | 1.1 |

As shown in Table 2, compared to the lithium batteries of Comparative Examples 1 to 4 that do not include the compound represented by Formula 1 and the compound represented by Formula 2, the lithium batteries of Examples 1 to 5 including the additive disclosed herein show improvements in room-temperature capacity retention ratio, high-temperature capacity retention ratio, high-temperature resistance change, and high-temperature gas generation amount.

[EXPLANATION OF REFERENCE NUMERALS DESIGNATING THE MAJOR ELEMENTS OF THE DRAWINGS]

| | | | |
|---|---|---|---|
| 1: | LITHIUM BATTERY | 2: | NEGATIVE ELECTRODE |
| 3: | POSITIVE ELECTRODE | 4: | SEPARATOR |
| 5: | BATTERY CASE | 6: | CAP ASSEMBLY |

## Claims

**1.** An additive for an electrolyte of a lithium battery, the additive comprising:
a compound represented by Formula 1 and a compound represented by Formula 2:

Formula 1

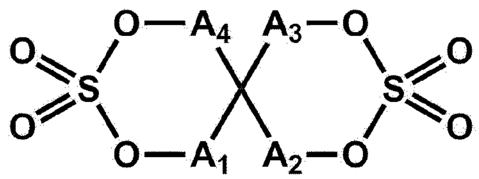

Formula 2

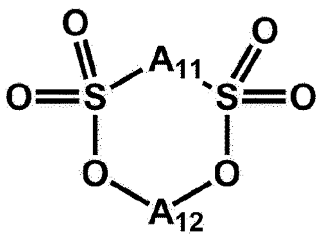

wherein in Formulas 1 and 2,
$A_1$, to $A_4$, and $A_{11}$ to $A_{12}$ are each independently a C1-C5 alkylene group unsubstituted or substituted with a substituent; a carbonyl group; or a sulfinyl group.

**2.** The additive of claim 1,

wherein at least one from among $A_1$ to $A_4$, $A_{11}$, and $A_{12}$ is an unsubstituted C1-C5 alkylene group or a substituted C1-C5 alkylene group,
wherein the substituent of the substituted C1-C5 alkylene group is a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C20 cycloalkenyl group unsubstituted or substituted with a halogen atom; a C3-C20 heterocyclyl group unsubstituted or substituted with a halogen atom; a C6-C40 aryl group unsubstituted or substituted with a halogen atom; a C2-C40 heteroaryl group unsubstituted or substituted with a halogen atom; or a polar functional group including a heteroatom.

**3.** The additive of claim 1,
wherein the substituent is at least one selected from among a halogen atom, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

**4.** The additive of claim 1,

wherein the substituted C1-C5 alkylene group is substituted with a polar functional group containing at least one heteroatom,
wherein the polar functional group containing the heteroatom comprises at least one selected from among -F, -Cl, -Br, -I, $-C(=O)OR^{16}$, $-OC(=O)R^{16}$, $-OR^{16}$, $-OC(=O)OR^{16}$, $-R^{15}OC(=O)OR^{16}$, $-C(=O)R^{16}$, $-R^{15}C(=O)R^{16}$, $-OC(=O)R^{16}$, $-R^{15}OC(=O)R^{16}$, $-(R^{15}O)_{k-}OR^{16}$, $-(OR^{15})_{k-}OR^{16}$, $-C(=O)-O-C(=O)R^{16}$, $-R^{15}C(=O)-O-C(=O)R^{16}$, $-SR^{16}$, $-R^{15}SR^{16}$, $-SSR^{16}$, $-R^{15}SSR^{16}$, $-S(=O)R^{16}$, $-R^{15}S(=O)R^{16}$, $-R^{15}C(=S)R^{16}$, $-R^{15}C(=S)SR^{16}$, $-R^{15}SO_3R^{16}$, $-SO_3R^{16}$, $-NNC(=S)R^{16}$, $-R^{15}NNC(=S)R^{16}$, $-R^{15}N=C=S$, -NCO, $-R^{15}-NCO$, $-NO_2$, $-R^{15}NO_2$, $-R^{15}SO_2R^{16}$, $-SO_2R^{16}$,

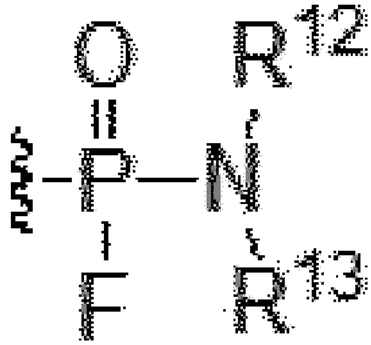
,
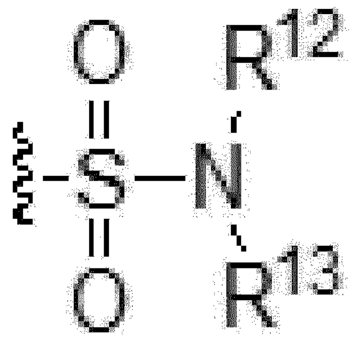
,

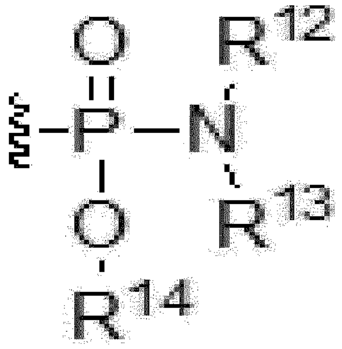
,
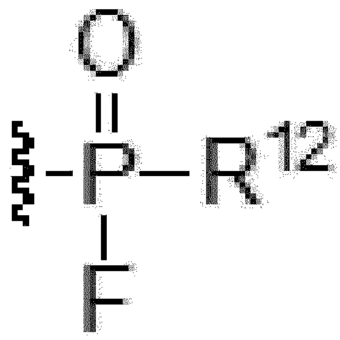
,
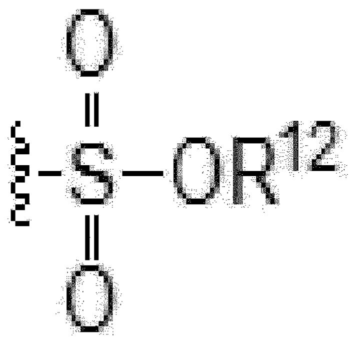
,

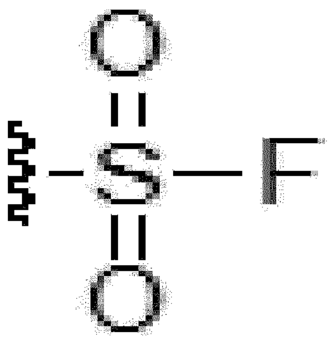 , 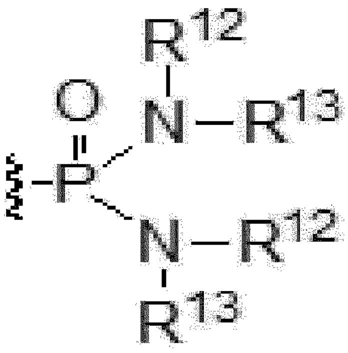 , 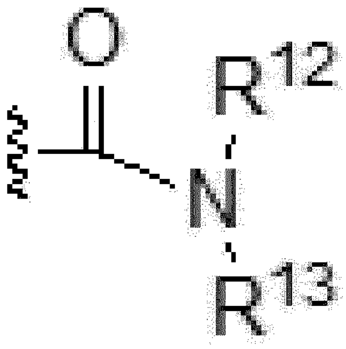 ,

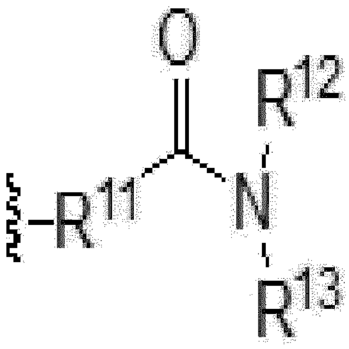 , 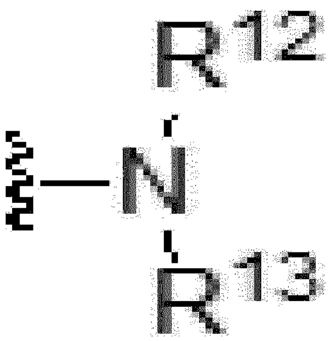 , 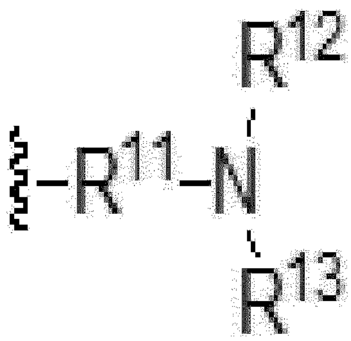 ,

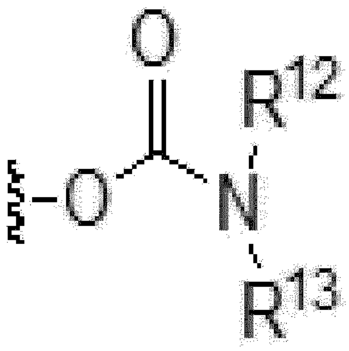 , 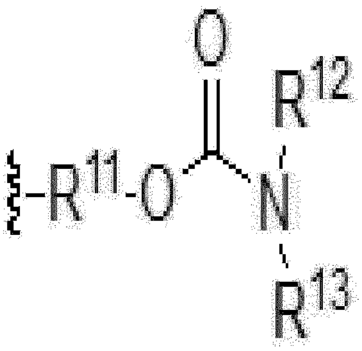 , 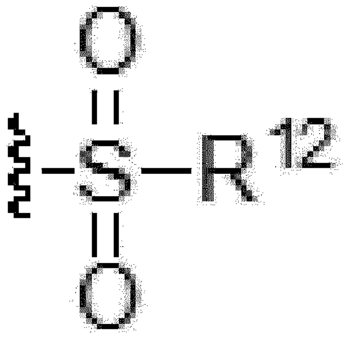 ,

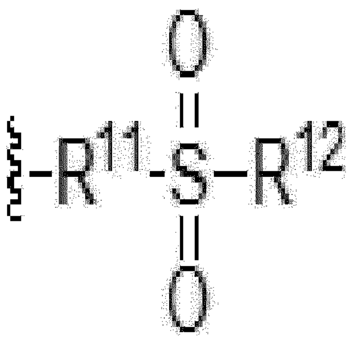 , 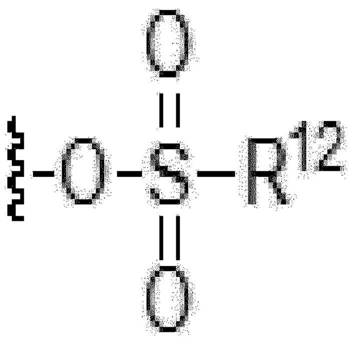 , 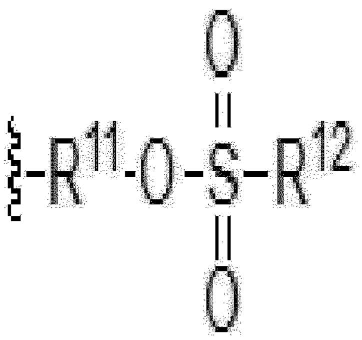 ,

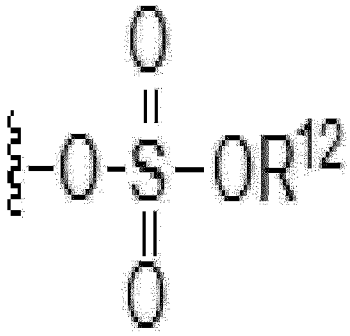 , 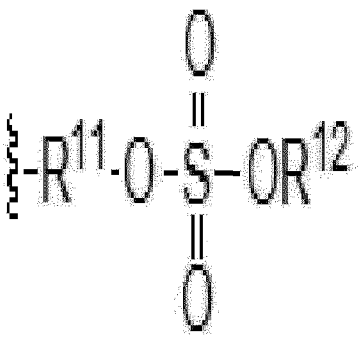 , 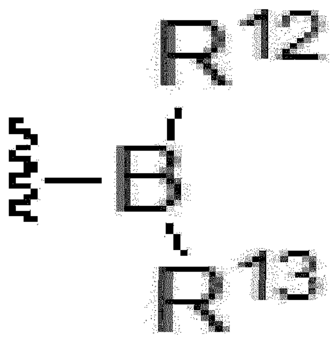 ,

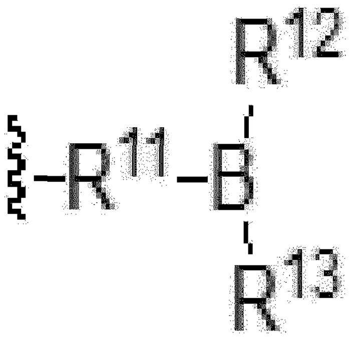 , 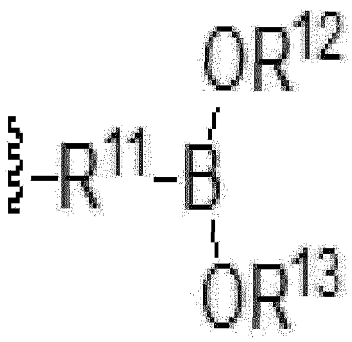 , 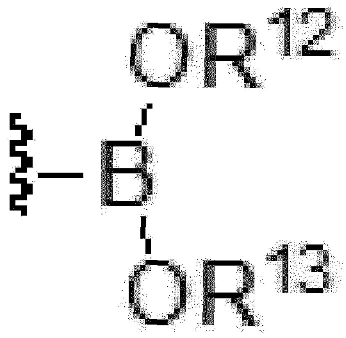 ,

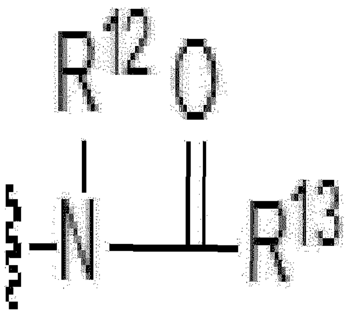 , 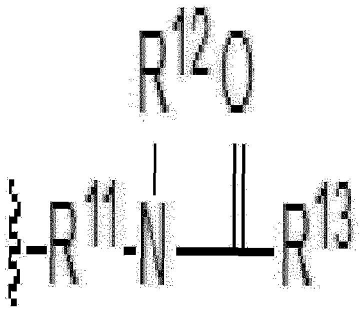 , 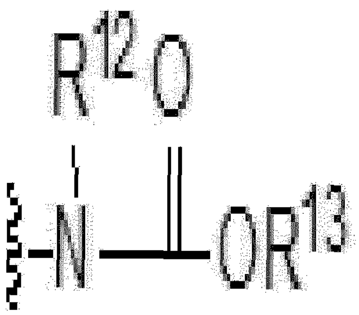 ,

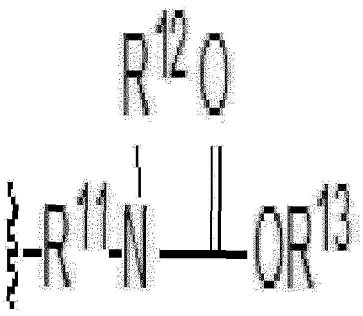 , 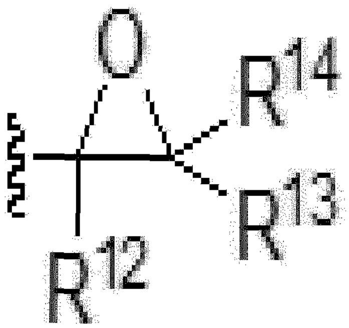 , 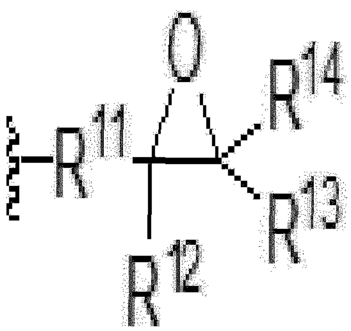 ,

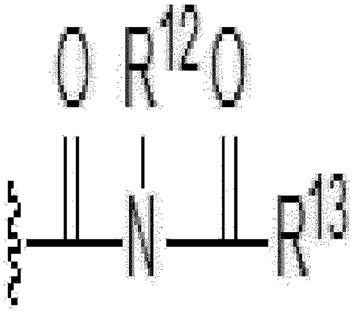,
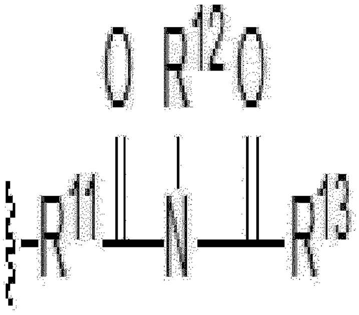,
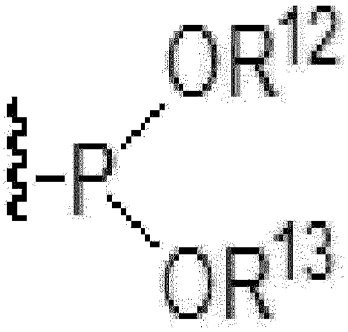,

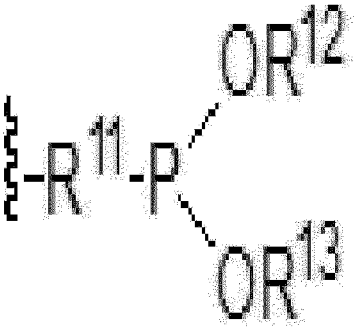,
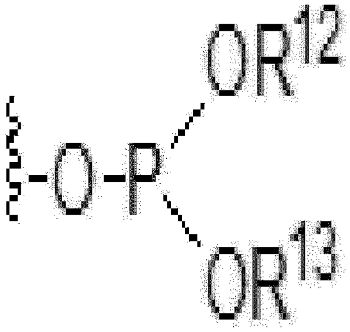,
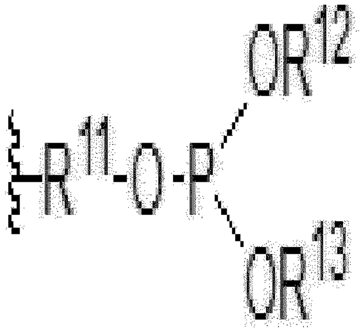,

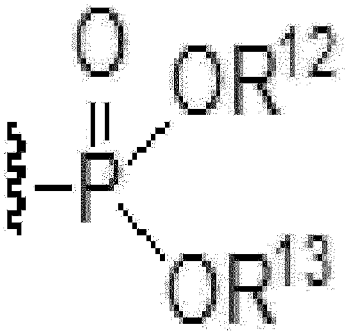,
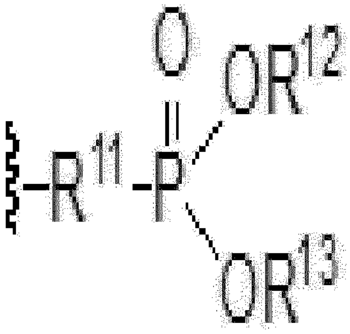,
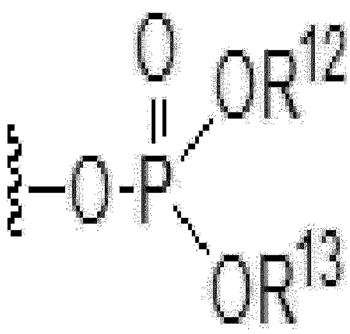,

and

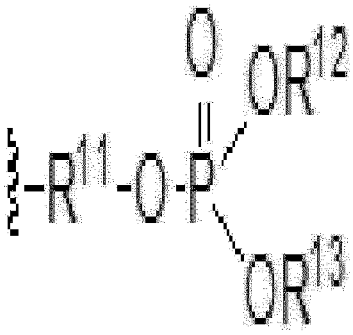,

$R^{11}$ and $R^{15}$ are each independently a C1-C20 alkylene group unsubstituted or substituted with a halogen atom; a C2-C20 alkenylene group unsubstituted or substituted with a halogen atom; a C2-C20 alkynylene group unsubstituted or substituted with a halogen atom; a C3-C12 cycloalkylene group unsubstituted or substituted with a halogen atom; a C6-C40 arylene group unsubstituted or substituted with a halogen atom; a C2-C40

heteroarylene group unsubstituted or substituted with a halogen atom; a C7-C15 alkylarylene group unsubstituted or substituted with a halogen atom; or a C7-C15 aralkylene group unsubstituted or substituted with a halogen atom,

$R^{12}$, $R^{13}$, $R^{14}$, and $R^{16}$ are each independently hydrogen; a halogen atom; a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C12 cycloalkyl group unsubstituted or substituted with a halogen atom; a C6-C40 aryl group unsubstituted or substituted with a halogen atom; a C2-C40 heteroaryl group unsubstituted or substituted with a halogen atom; a C7-C15 alkylaryl group unsubstituted or substituted with a halogen atom; a C7-C15 trialkylsilyl group unsubstituted or substituted with a halogen atom; or a C7-C15 aralkyl group unsubstituted or substituted with a halogen atom, and

k is an integer of 1 to 20.

**5.** The additive of claim 1,
wherein the compound represented by Formula 1 is represented by Formula 1-1, and the compound represented by Formula 2 is represented by Formula 2-1:

Formula 1-1

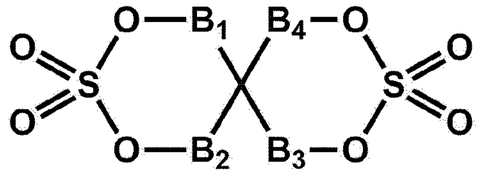

Formula 2-1

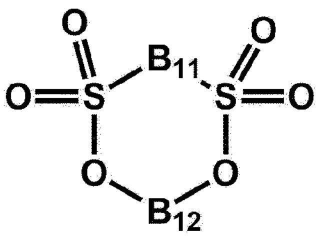

wherein in Formula 1-1 and Formula 2-1,

$B_1$, $B_2$, $B_3$, $B_4$, $B_{11}$, and $B_{12}$ are each independently -C($E_1$)($E_2$)-; a carbonyl group; or a sulfinyl group,
wherein $E_1$ and $E_2$ are each independently hydrogen; a halogen atom; a C1-C20 alkyl group unsubstituted or substituted with a halogen atom; a C2-C20 alkenyl group unsubstituted or substituted with a halogen atom; a C2-C20 alkynyl group unsubstituted or substituted with a halogen atom; a C3-C20 cycloalkenyl group unsubstituted or substituted with a halogen atom; a C3-C20 heterocyclyl group unsubstituted or substituted with a halogen atom; a C6-C40 aryl group unsubstituted or substituted with a halogen atom; or a C2-C40 heteroaryl group unsubstituted or substituted with a halogen atom.

**6.** The additive of claim 5,
wherein $E_1$ and $E_2$ are each independently hydrogen; a halogen atom; a C1-C10 alkyl group unsubstituted or substituted with a halogen atom; a C6-C40 aryl group unsubstituted or substituted with a halogen atom; or a C2-C40 heteroaryl group unsubstituted or substituted with a halogen atom.

**7.** The additive of claim 5,
wherein $E_1$ and $E_2$ are each independently selected from among hydrogen, F, Cl, Br, I, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

**8.** The additive of claim 1,

wherein the compound represented by Formula 1 is represented by Formula 1-2, and the compound represented by Formula 2 is represented by Formula 2-2:

Formula 1-2

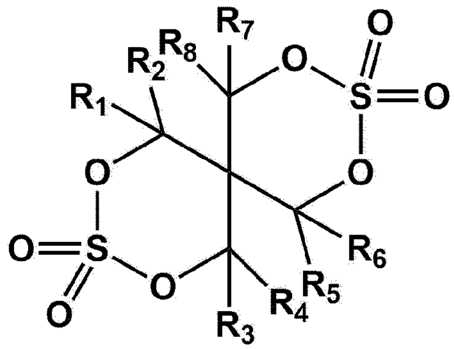

Formula 2-2

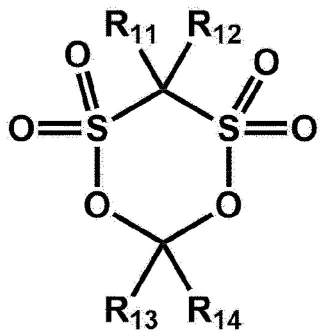

wherein in Formula 1-2 and Formula 2-2,
$R_1$ to $R_8$ and $R_{11}$ to $R_{14}$ are each independently hydrogen; a halogen atom; a C1-C20 alkyl group unsubstituted or substituted with a halogen atom; a C6-C40 aryl group unsubstituted or substituted with a halogen atom; or a C2-C40 heteroaryl group unsubstituted or substituted with a halogen atom.

**9.** The additive of claim 8,
wherein $R_1$ to $R_8$ and $R_{11}$ to $R_{14}$ are each independently selected from among hydrogen, F, Cl, Br, I, a methyl group, an ethyl group, a propyl group, an isopropyl group, a butyl group, a tert-butyl group, a trifluoromethyl group, a tetrafluoroethyl group, a phenyl group, a naphthyl group, a tetrafluorophenyl group, a pyrrolyl group, and a pyridinyl group.

**10.** The additive of claim 1,
wherein the compound represented by Formula 1 is represented by one selected from among Formula 1-3 to Formula 1-11:

Formula 1-3

Formula 1-4

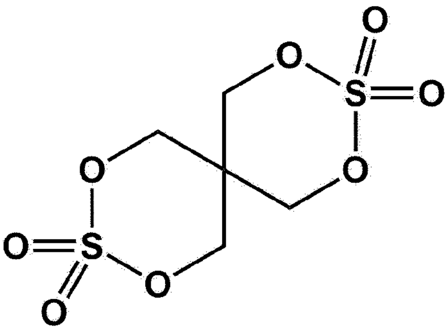

Formula 1-5

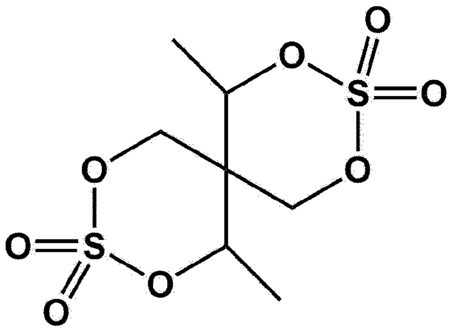

Formula 1-6

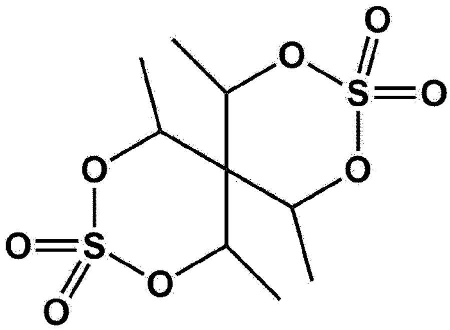

Formula 1-7

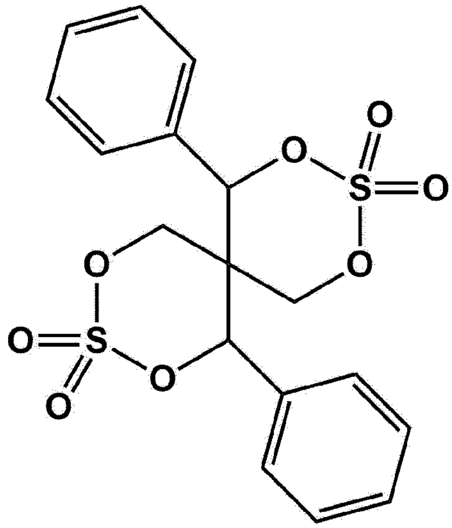

Formula 1-8

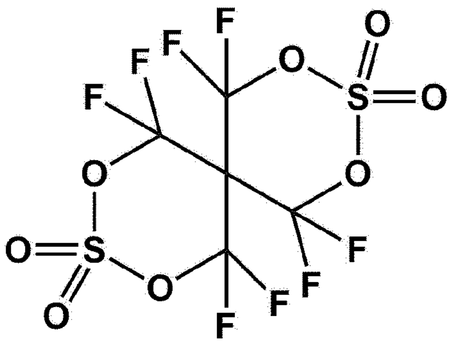

Formula 1-9

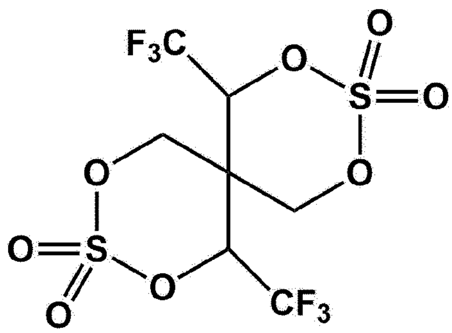

Formula 1-10

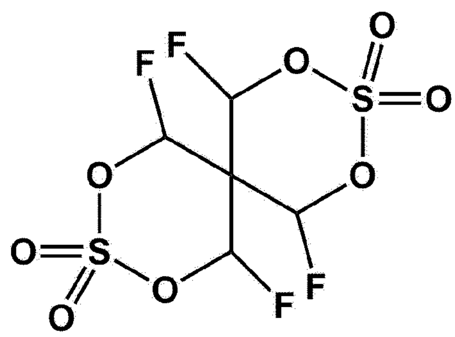

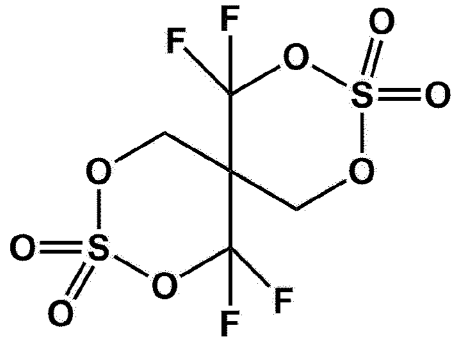

Formula 1-11

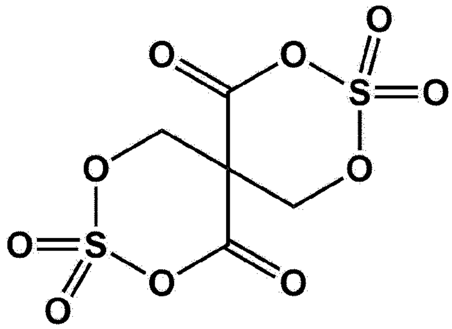

**11.** The additive of claim 1, wherein the compound represented by Formula 2 is represented by one selected from among Formula 2-3 to Formula 2-12:

Formula 2-3

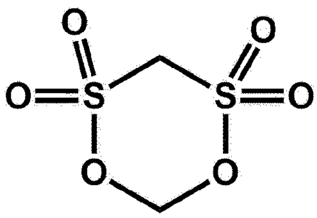

Formula 2-4

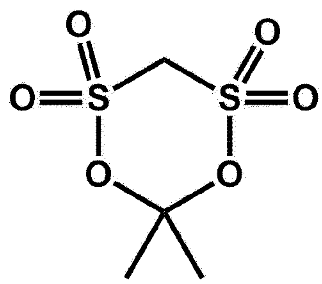

Formula 2-5

Formula 2-6

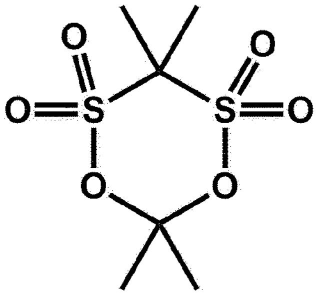

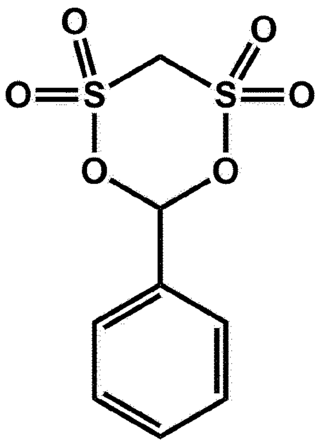

Formula 2-7

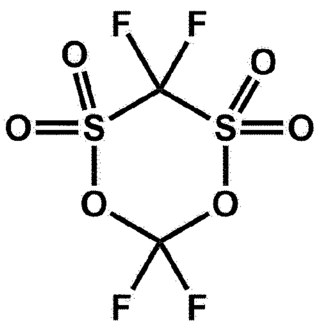

Formula 2-8

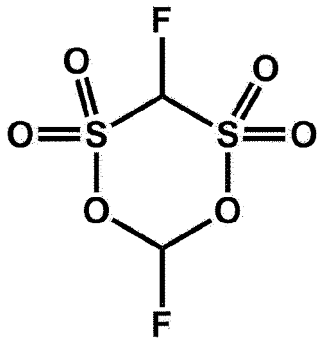

Formula 2-9

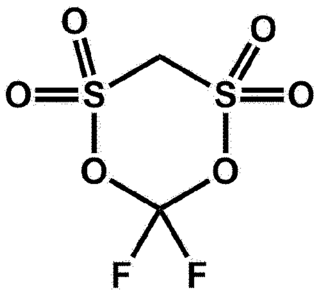

Formula 2-10

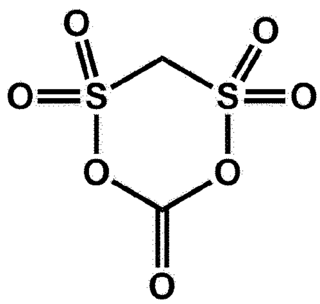

Formula 2-11

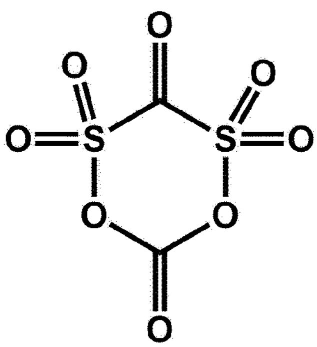

Formula 2-12

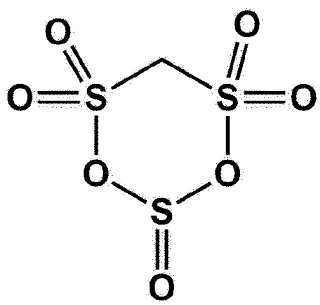

**12.** An organic electrolyte comprising:

a first lithium salt;
an organic solvent; and
the additive according to claim 1.

**13.** The organic electrolyte of claim 12,
wherein a content of the compound represented by Formula 1 is 0.1 wt% to 5.0 wt% with respect to a total weight of the organic electrolyte.

**14.** The organic electrolyte of claim 12,
wherein a content of the compound represented by Formula 2 is 0.1 wt% to 5.0 wt% with respect to a total weight of the organic electrolyte.

**15.** The organic electrolyte of claim 12,
wherein a ratio of a content of the compound represented by Formula 2 to a content of the compound represented by Formula 1 is from 1:10 to 10:1.

**16.** A lithium battery comprising:

a positive electrode; a negative electrode; and
the organic electrolyte according to claim 12.

**17.** The lithium battery of claim 16,
wherein the positive electrode comprises a nickel-containing layered-structure lithium transition metal oxide.

**18.** The lithium battery of claim 17,
wherein the lithium transition metal oxide comprises at least one from among oxides represented by Formula 12 to Formula 14:

Formula 12 $LiNi_xCo_yMn_zO_2$

Formula 13 $LiNi_xCo_yAl_zO_2$

wherein in Formulas 12 and 13,
$0.6 \leq x \leq 0.95$, $0 < y \leq 0.2$, $0 < z \leq 0.2$, and $x+y+z=1$,

Formula 14 $LiNi_xCo_yMn_xAl_wO_2$

wherein in Formula 14,
$0.6 \leq x \leq 0.95$, $0 < y \leq 0.2$, $0 < z \leq 0.2$, $0 < w \leq 0.2$, and $x+y+z+w=1$.

FIG. 1

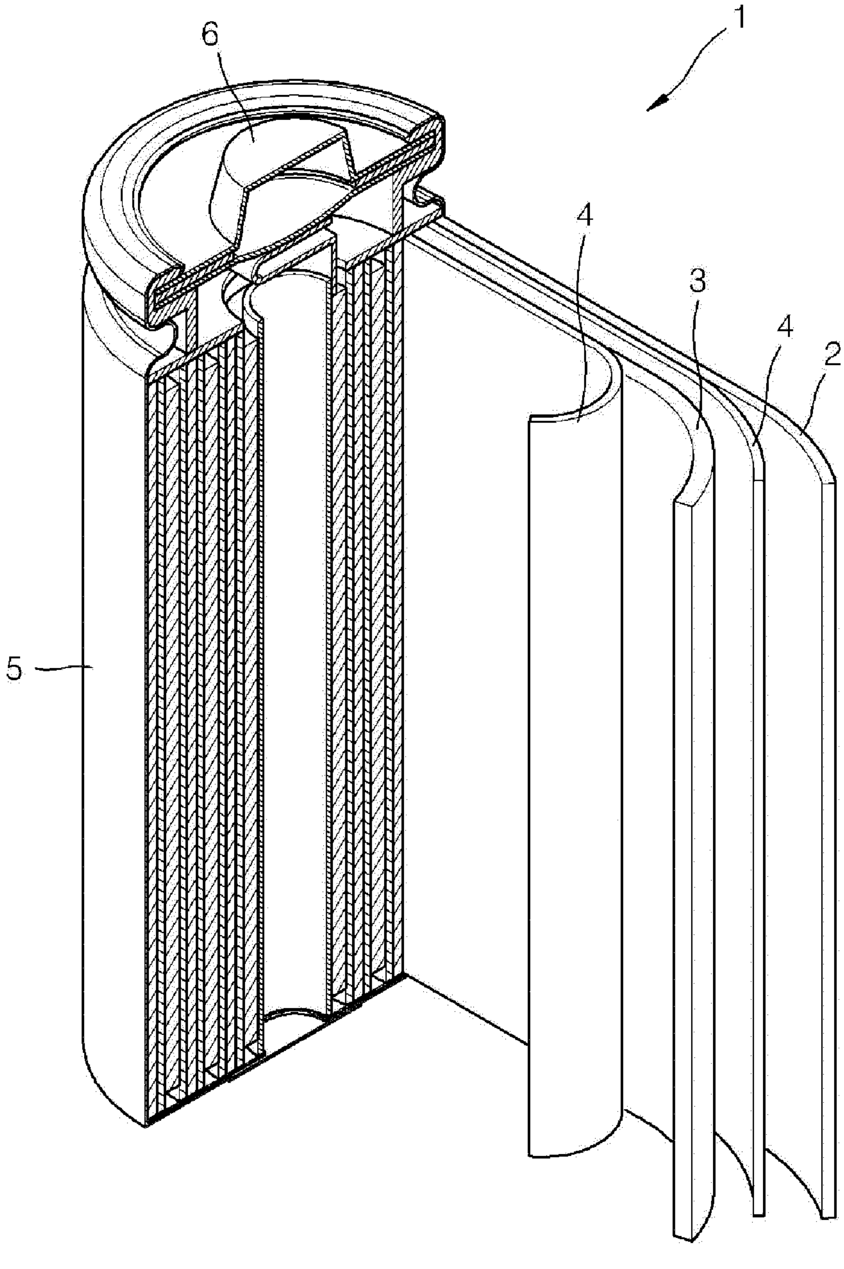

**INTERNATIONAL SEARCH REPORT**

International application No.
**PCT/KR2021/020273**

**A. CLASSIFICATION OF SUBJECT MATTER**

**H01M 10/0567**(2010.01)i; **H01M 4/525**(2010.01)i; **H01M 4/505**(2010.01)i; **H01M 10/052**(2010.01)i

According to International Patent Classification (IPC) or to both national classification and IPC

**B. FIELDS SEARCHED**

Minimum documentation searched (classification system followed by classification symbols)

H01M 10/0567(2010.01); H01M 10/052(2010.01); H01M 10/0525(2010.01); H01M 10/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 펜타에리트리톨 디설페이트(pentaerythritol disulfate), 메틸렌 메탄 디설포네이트(methylene methane disulfonate), 리튬 전지(lithium battery), 전해액(electrolyte)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2019-0080040 A (PANAX ETEC CO., LTD.) 08 July 2019 (2019-07-08)<br>See claims 1, 10 and 11; and paragraphs [0022], [0051], [0054] and [0096]. | 1,2,5-18<br>3,4 |
| Y | US 2020-0144669 A1 (SHENZHEN CAPCHEM TECHNOLOGY CO., LTD.) 07 May 2020 (2020-05-07)<br>See claims 1-10; and paragraph [0030]. | 3,4 |
| A | KR 10-2018-0136655 A (SK CHEMICALS CO., LTD.) 26 December 2018 (2018-12-26)<br>See claims 1, 5 and 7; and paragraphs [0027], [0031], [0032], [0040], [0041] and [0053]. | 1-18 |
| A | KR 10-2019-0054973 A (LG CHEM, LTD.) 22 May 2019 (2019-05-22)<br>See entire document. | 1-18 |
| A | KR 10-1944226 B1 (NISSAN MOTOR CO., LTD.) 30 January 2019 (2019-01-30)<br>See entire document. | 1-18 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2022** | **11 April 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**
**Information on patent family members**

International application No.
**PCT/KR2021/020273**

| Patent document cited in search report | Publication date (day/month/year) | Patent family member(s) | Publication date (day/month/year) |
|---|---|---|---|
| KR 10-2019-0080040 A | 08 July 2019 | None | |
| US 2020-0144669 A1 | 07 May 2020 | CN 109326824 A | 12 February 2019 |
| | | CN 109326824 B | 21 April 2020 |
| | | EP 3664212 A1 | 10 June 2020 |
| | | EP 3664212 B1 | 12 May 2021 |
| | | WO 2019-024412 A1 | 07 February 2019 |
| KR 10-2018-0136655 A | 26 December 2018 | None | |
| KR 10-2019-0054973 A | 22 May 2019 | CN 111052485 A | 21 April 2020 |
| | | EP 3648231 A1 | 06 May 2020 |
| | | EP 3648231 B1 | 07 July 2021 |
| | | JP 2020-528639 A | 24 September 2020 |
| | | US 2020-0220216 A1 | 09 July 2020 |
| | | WO 2019-093853 A1 | 16 May 2019 |
| KR 10-1944226 B1 | 30 January 2019 | CN 107851832 A | 27 March 2018 |
| | | CN 107851832 B | 09 June 2020 |
| | | EP 3322023 A1 | 16 May 2018 |
| | | EP 3322023 B1 | 28 October 2020 |
| | | JP 6575972 B2 | 18 September 2019 |
| | | US 10270127 B2 | 23 April 2019 |
| | | US 2018-0191027 A1 | 05 July 2018 |
| | | WO 2017-006480 A1 | 12 January 2017 |

Form PCT/ISA/210 (patent family annex) (July 2019)